# EUROPÄISCHE PATENTSCHRIFT

(11) **EP 1 587 419 B1**
(45) Veröffentlichungstag und Bekanntmachung des Hinweises auf die Patenterteilung: **18.11.2009**
(21) Anmeldenummer: 03779533.3
(22) Anmeldetag: 19.11.2003
(51) Int. Cl.: A61B 5/15, A61M 5/32

(54) **MEDIZINISCHE BAUGRUPPE SOWIE EINE SCHUTZVORRICHTUNG, EIN EINSTICHELEMENT UND EINE HANDHABUNGSVORRICHTUNG FÜR DIESE BAUGRUPPE**
MEDICAL ASSEMBLY, IN ADDITION TO A GUARD DEVICE, PUNCTURE ELEMENT AND A MANIPULATION DEVICE FOR SAID ASSEMBLY
ENSEMBLE MEDICAL AVEC DISPOSITIF DE PROTECTION, ELEMENT DE PIQURE ET DISPOSITIF DE MANIPULATION ASSOCIES

(30) Priorität: 27.01.2003 AT 1042003
(43) Veröffentlichungstag der Anmeldung: 26.10.2005
(73) Patentinhaber: Greiner Bio-One GmbH, 4550 Kremsmünster (AT)
(72) Erfinder: KONRAD, Franz, A-4690 Oberndorf bei Schwanenstadt (AT)
(74) Vertreter: Burger, Hannes
(86) Internationale Anmeldenummer: PCT/AT2003/000345
(87) Internationale Veröffentlichungsnummer: WO 2004/066840

(56) Entgegenhaltungen:
- EP-A- 0 812 597
- WO-A-03/006082
- US-A- 5 139 489
- US-A- 5 277 311
- US-A- 5 312 369
- US-A1- 2002 156 424
- US-A1- 2002 156 427
- US-A1- 2002 193 744

## Beschreibung

Die Erfindung bezieht sich auf eine Baugruppe, insbesondere für die Medizintechnik, umfassend eine Handhabungsvorrichtung mit einem daran angeordneten ersten Halteelement sowie einem ersten Kupplungselement, ein Einstichelement mit einem proximalen sowie einem distalen Ende, die in Richtung einer Längsachse voneinander distanziert sind, wobei ein Durchgang die beiden Enden miteinander verbindet, und einem zwischen den Enden angeordneten weiteren Kupplungselement, wobei die beiden Kupplungselemente eine Kupplungsvorrichtung ausbilden und das Einstichelement an der Handhabungsvorrichtung bedarfsweise lösbar gehalten ist, wobei das proximale Ende des Einstichelements die Handhabungsvorrichtung überragt und zumindest am proximalen Ende eine Öffnung mit einer längeren und kürzeren Öffnungsachse angeordnet ist, eine schwenkbare Schutzvorrichtung für zumindest einen Teilbereich des Einstichelementes, und die Schutzvorrichtung bedarfsweise lösbar mit einem weiteren Halteelement feststehend am ersten Halteelement der Handhabungsvorrichtung gehalten ist, wobei die beiden Halteelemente eine Haltevorrichtung ausbilden und ein Schutzelement der Schutzvorrichtung in einer in der Längsachse verlaufend ausgerichteten Schwenkebene von einer ersten, den Teilbereich des Einstichelements freigebenden Stellung (Freigabestellung) in eine zweite, den Teilbereich abdeckenden Stellung (Schutzstellung) verschwenkbar ist, wobei zwischen den beiden Halteelementen der Haltevorrichtung eine Positioniervorrichtung angeordnet ist und diese bei in Eingriff stehenden Halteelementen die Schwenkebene des Schutzelementes der Schutzvorrichtung in Bezug zur Handhabungsvorrichtung in ihrer Lage festlegt und dass die beiden Kupplungselemente der Kupplungsvorrichtung zueinander derart ausgebildet sind, dass bei vollständig gekuppelter Stellung der beiden Kupplungselemente die kürzere Öffnungsachse der Öffnung etwa parallel zur Schwenkebene oder in der Schwenkebene verlaufend ausgerichtet ist.

Aus der US 5,139,489 A ist ein Gehäusehalter zur Aufnahme eines Aufnahmeröhrchens bekannt geworden, welcher an einer vom Innenraum abgewendeten Seite einen Fortsatz mit einem daran angeordneten Außen- sowie Innengewinde aufweist. Mit dem Außengewinde des Fortsatzes ist ein Basisteil der schwenkbar ausgebildeten Schutzvorrichtung mittels eines darin angeordneten Innengewindes aufschraubbar. Dieser Basisteil weist weiters in dessen Zentrum einen Durchbruch auf, der in seiner Lage mit dem im Fortsatz angeordneten Innengewinde lagemäßig korrespondiert. In das Innengewinde des Fortsatzes des Gehäusehalters ist die Nadelanordnung einschraubbar. Zu diesem Zweck ist das Schutzgehäuse um eine Schwenkachse ausgehend von der durch den Gehäusehalter verlaufenden Längsachse wegzuschwenken, um diesen Einschraubvorgang durchführen zu können. Eine exakt vorbestimmbare Ausrichtung des Nadelendes in Bezug auf die Schwenkebene des Schutzgehäuses konnte dabei nicht immer sichergestellt werden.

Aus der EP 0 812 597 A2 sind andere Ausbildungsmöglichkeiten einer Schutzvorrichtung für eine Nadelanordnung bekannt geworden, bei welcher entweder der Basisteil der Schutzvorrichtung mittels der Nadelanordnung schwenkbar um die Längsachse des Halters an diesem gehalten oder der Basisteil des Schutzelements auf die Nadelanordnung aufgesetzt bzw. auch mit dieser verbunden ist. Die so geschaffene Einheit ist über einen Normanschluss mit einem Spritzengehäuse oder einem Gehäusehalter für Blutentnahmeröhrchen verbindbar.

Aus der US 5,277,311 A sowie der US 5,312,369 A sind jeweils Schutzvorrichtungen für Nadelanordnungen bekannt geworden, bei welchen der Basiskörper des schwenkbar ausgebildeten Schutzelements der Schutzvorrichtung dreh- bzw. schwenkbar am Fortsatz des Gehäusehalters bzw. der Handhabungsvorrichtung angeordnet ist. Zusätzlich können dabei zwischen dem Fortsatz und dem Basisteil reibungserhöhende Bauteile vorgesehen sein, um die aufzubringende Kraft festzulegen, welche notwendig ist, um den Basisteil der Schutzvorrichtung relativ zur Längsachse um den Fortsatz verdrehen zu können. Das Schutzelement ist über verformbar ausgebildete Teile bzw. Gelenke von einer Freigabestellung der Nadelanordnung hin in eine Schutz- bzw. Abdeckstellung verschwenkbar.

Aus der US 3,658,061 A ist eine medizinische Baugruppe, beispielsweise für die Blutabnahme, bekannt geworden, die ein Handhabungsvorrichtung, ein daran gehaltertes bzw. eingesetztes Einstichelement sowie eine schwenkbare Schutzvorrichtung für zumindest einen Teilbereich des Einstichelements umfasst. Die Schutzvorrichtung ist bedarfsweise lösbar, jedoch feststehend an der Handhabungsvorrichtung gehaltert, wobei ein flexibler Teil zwischen dem Schutzelement und dem Halteelement der Schutzvorrichtung eine Gelenkanordnung ausbildet. Die Schutzvorrichtung ist von einer ersten, das Einstichelement freigebenden, in eine zweite, das Einstichelement abdeckende Stellung verschwenkbar. Das Schutzelement weist einen Kanal zur Aufnahme eines Teilbereiches des Einstichelements auf, wobei dieser Kanal derart ausgebildet ist, dass mittels Reibschluss bzw. Klemmung das Schutzelement in der Schutzstellung am Einstichelement gehalten ist.

Ein weiteres Schutzelement ist aus der US 4,664,259 A bekannt geworden, bei welchem im Bereich des Schutzelements in der eingeklappten Stellung, also der Schutzstellung, dem Schutzelement ein Rastelement zugeordnet ist, mit welchem das Schutzelement in der Schutzstellung am Einstichelement, insbesondere der Hohlnadel, verrastet gehalten werden kann, um so ein unbeabsichtigtes, neuerliches Freigeben des proximalen Endes des Einstichelementes zu verhindern bzw. zu vermeiden.

Aus der EP 0 626 924 B1 ist eine andere Schutzvorrichtung für eine medizinische Baugruppe bekannt geworden, bei der das Schutzelement bedarfsweise lösbar an der Handhabungsvorrichtung anbringbar ist. Dabei ist das Halteelement der Schutzvorrichtung in Richtung der Längsachse, also in axialer Richtung der Handhabungsvorrichtung feststehend an dieser gehaltert, wobei jedoch eine Drehung des Halteelements mitsamt dem Schutzelement um die Längsachse möglich ist.

Weitere schwenkbare Nadelschutzvorrichtungen sind aus der EP 0 702 973 B1 bzw. der EP 0 885 621 B1 bekannt geworden, bei welchen jeweils das Halteelement für das Schutzelement der Schutzvorrichtung an einem von der Handhabungsvorrichtung abgewendeten Bereich des Einstichelementes an diesem gehalten ist. Dadurch wird eine Baueinheit geschaffen, bei welcher das Einstichelement zumeist gemeinsam mit dem Schutzelement in die Handhabungsvorrichtung einzusetzen ist. Ähnlich ausgestaltete Schutzvorrichtungen sind aber auch noch aus der US 6,436,086 B1, US 6,440,104 B1, US 2002/0151852 A1, US 2002/0151853 A1, US 2002/0156425 As, US 2002/0156427 As, US 2002/0161336 A1 sowie der US 2002/0193744 A1 bekannt geworden.

Nachteilig bei all diesen Nadelschutzvorrichtungen ist, dass die Lage der Öffnung am proximalen Ende stets unbestimmt zur vorgegebenen Lage der Schwenkebene des Schutzelementes war, wie dies bei den voneinander unabhängig an der Handhabungsvorrichtung anbringbaren Bauteilen, nämlich der Schutzvorrichtung sowie dem Einstichelement der Fall war.

Der vorliegenden Erfindung liegt die Aufgabe zugrunde, eine Baugruppe, insbesondere für die Medizintechnik, zu schaffen, die unabhängig voneinander an der Handhabungsvorrichtung anbringbar sind und mit welcher eine sichere und vor allem störungsfreie Bedienung während des bestimmungsgemäßen Gebrauches möglich ist.

Diese Aufgabe der Erfindung wird durch die Gesamtheit der Merkmale des Anspruches 1, insbesondere dadurch gelöst, dass die Positioniervorrichtung im Bereich der Handhabungsvorrichtung durch zumindest ein erstes in Richtung der Längsachse sowie senkrecht zu dieser ausgerichtetes Positionierelement und im Bereich des weiteren Halteelementes durch zumindest ein weiteres dazu in etwa gegengleich ausgebildetes Positionierelement gebildet ist. Der sich daraus ergebende überraschende Vorteil ergibt sich dadurch, dass durch die zwischen den beiden Halteelementen der Haltevorrichtung angeordnete Positioniervorrichtung mit deren Positionierelementen und bei in Eingriff stehenden Halteelementen auf einfache Art und Weise die Ausrichtung und damit verbunden die Lage der Schwenkebene in Bezug zur Handhabungsvorrichtung festlegbar ist. Dadurch wird nicht nur eine feststehende Lage der gesamten Schutzvorrichtung an der Handhabungsvorrichtung; sondern auch eine exakte, vorbestimmbare Ausrichtung der Schwenkebene erzielt. Durch die zusätzliche Anordnung der Positioniervorrichtung wird eine definierte Lage des Schutzelements relativ zur Handhabungsvorrichtung erzielt. Darüber hinaus wird durch die Ausbildung der Kupplungselemente zwischen dem Einstichelement und der Handhabungsvorrichtung die Lage und somit die Ausrichtung der Öffnung im Bereich des proximalen Endes in Abhängigkeit von der Positioniervorrichtung derart festgelegt, dass die kurze Öffnungsachse der Öffnung etwa parallel zur Schwenkebene oder in der Schwenkebene verlaufend ausgerichtet ist. Dadurch wird erreicht, dass bei bestimmungsgemäßer Verwendung der Baugruppe, beispielsweise beim Aufsammeln bzw. Abnehmen von Körperflüssigkeiten bzw. der Abgabe von Fluiden in den Körper eines Patienten, das Schutzelement stets seitlich in bezug auf die Handhabungsvorrichtung angeordnet ist. Dadurch wird eine Verwendung der Baugruppe, insbesondere für die Medizintechnik, mit hoher Anwendersicherheit erzielt, da aufgrund der Lageabstimmung zwischen der Schwenkebene im Zusammenwirken mit der Positioniervorrichtung und der daraufhin abgestimmten Ausbildung der Kupplungsvorrichtung stets vermieden wird, und das Schutzelement entweder dem Patienten direkt zugewandt oder der Bedienperson ein Anblick der Öffnung am proximalen Ende erschwert bzw. verhindert wird. Durch die vorbestimmte seitliche Anordnung des Schutzelementes kann auch noch eine einfache Schließbewegung des Schutzelementes hin in die Schutzstellung dadurch erfolgen, dass das Schutzelement in seiner Freigabestellung z.B. nur gegen einen entsprechend stabilen Gegenstand gedrückt werden muss und dadurch ohne jeglicher Verletzungsgefahr das proximale Ende des Einstichelementes abgedeckt werden kann. Weiters wird damit aber auch eine einfache Einsetzbewegung des Einstichelementes in die Handhabungsvorrichtung erzielt, da die Kanüle ohne jegliches Beiwerk, wie die schwenkbare Schutzvorrichtung, einfach angekuppelt werden kann.

Vorteilhaft ist auch eine weitere Ausführungsform nach Anspruch 2, da dadurch eine leicht handhabbare Handhabungsvorrichtung geschaffen wird, die die Aufnahme entsprechend ausgestalteter Aufnahmegefäß, wie beispielsweise Blutprobenröhrchen, begünstigt.

Vorteilhaft ist weiters eine Ausbildung nach Anspruch 3, da dadurch auf geringstem Raum eine einfache und vor allem sichere Halterung bzw. Kupplung unterschiedlicher Bauelemente an der Handhabungsvorrichtung ermöglicht wird.

Durch die Ausbildung nach Anspruch 4 oder 5 ist es möglich, an der Handhabungsvorrichtung einen einfach ausgestalteten und zu entformenden Bauteil auszubilden, an welchem sowohl das Einstichelement als auch die schwenkbare Schutzvorrichtung einfach gehaltert werden kann.

Nach einer anderen Ausführungsvariante gemäß Anspruch 6 wird auf einfache Art und Weise durch die beiderseitige Ausbildung der rohrförmigen Bauteile eine Halterung der Schutzvorrichtung an der Handhabungsvorrichtung erzielt.

Vorteilhaft sind aber auch Weiterbildungen nach den Ansprüchen 7 bis 9, weil dadurch eine axiale Fixierung bzw. Halterung des Halteelements der Schutzvorrichtung am Halteelement der Handhabungsvorrichtung sichergestellt wird und so eine einfache, bedarfsweise anbringbare bzw. lösbare Halterung der Schutzvorrichtung an der Handhabungsvorrichtung erzielt.

Bei der Ausgestaltung nach Anspruch 10 ist von Vorteil, dass dadurch ein eindeutig festgelegter Schwenkmittelpunkt für das Schutzelement in bezug auf das an der Handhabungsvorrichtung gehaltene Einstichelement geschaffen wird.

Von Vorteil ist aber auch eine Ausbildung nach Anspruch 11, da dadurch eine kostengünstige Ausbildung für die Schwenkachse bei gleichzeitiger, eindeutiger Festlegung des Schwenkpunktes für das Schutzelement geschaffen wird.

Vorteilhaft ist die Ausbildung nach Anspruch 12, da so vor der ersten Verstellung eine gewisse Lagepositionierung des Schutzelementes relativ zur Stellung der Entnahmenadel vorbestimmt ist, wodurch eine sichere Bedienung ermöglicht wird.

Durch die Weiterbildung nach Anspruch 13 oder 14 wird erreicht, dass dadurch eine leicht zu bedienende Kupplungsvorrichtung geschaffen wird, bei welcher bedingt durch das doppelgängige Gewinde eine um 180° verschwenkte Endstellung der am proximalen Ende angeordneten Öffnung in bezug zur Handhabungsvorrichtung erzielbar ist und so einfach damit die Freigabestellung des Schutzelements während dem bestimmungsgemäßen Gebrauch festgelegt wird.

Durch die Ausbildung nach Anspruch 15 wird eine zusätzliche Lagefixierung des Einstichelements an der Handhabungsvorrichtung in Verbindung mit dem eingesetzten Einstichelement erzielt.

Vorteilhaft ist auch eine Ausbildung nach den Ansprüchen 16 bis 18, da dadurch auf einfache Art und Weise die Ausrichtung und damit verbunden die Lage der Schwenkebene in bezug zur Handhabungsvorrichtung festlegbar ist. Dadurch wird nicht nur eine feststehende Lage der gesamten Schutzvorrichtung an der Handhabungsvorrichtung, sondern auch eine exakte, vorbestimmbare Ausrichtung der Schwenkebene erzielt.

Dabei erweist sich eine Ausgestaltung nach Anspruch 19 vorteilhaft, da so in der Schutzstellung ein unbeabsichtigtes Lösen bzw. wiederum Freigeben des proximalen Endes des Einstichelements gesichert verhindert ist.

Bei der Ausgestaltung nach Anspruch 20 oder 21 ist von Vorteil, dass durch den im Schutzelement ausgebildeten Kanal das Einstichelement bis auf die notwendige Aufnahmeöffnung rund um vollständig abgedeckt ist und so eine hohe Sicherheit für das Bedienpersonal in der Schutzstellung erzielbar ist.

Nach einer anderen Ausführungsvariante gemäß Anspruch 22 wird das Schutzelement in deren Schutzstellung für das Einstichelement gegen ein unbeabsichtigtes Lösen arretiert gehalten, wodurch eine neuerliche Benutzung und damit verbunden entweder eine Verletzung für das Bedienpersonal bzw. auch damit eine Ansteckung von weiteren Patienten verhindert ist.

Vorteilhaft ist aber auch eine Ausbildung nach Anspruch 23 oder 24, da so durch die benachbarte Anordnung des Rückhalteelementes in Bezug zum Halteelement bzw. zu der daran angeordneten Schwenkanordnung, wie beispielsweise das Filmscharnier, der Abstand so gewählt ist, dass bei einem neuerlichen Öffnungsversuch ein Verbiegen des Einstichelementes aufgrund der kürzeren Hebelwirkung zwischen dem Rückhalteelement und der Einspannstelle desselben großteils verhindert ist. Bei einem größeren Abstand bzw. einer größeren Distanzierung des Rückhalteelementes vom Halteelement bzw. der Schwenkachse des Schutzelementes ist aufgrund der dem Einstichelement innewohnenden Elastizität ein Herausschlupfen aus dem Rückhalteelement möglich, wodurch ansonst auf einfache Art und Weise eine neuerliche Verwendung möglich wäre.

Vorteilhaft ist weiters auch eine Ausbildung nach Anspruch 25 oder 26, da so ein hoher Abdeckgrad und eine damit verbundene hohe Sicherheit für das Schutzelement erzielbar ist. Durch die Ausbildung nach Anspruch 27 ist es möglich, auch das weitere Kupplungselement des Einstichelementes von den Stegen des Schutzelementes abzudecken, wodurch auch in diesem Bereich ein hoher Überdeckungsgrad und damit verbunden eine hohe Sicherheit gegen Übertragung von Infektionen ermöglicht ist.

Nach einer anderen Auswhrungsvariante gemäß Anspruch 28 wird eine hohe Festigkeit und damit verbundene Eigensteifigkeit des Schutzelementes bei einem gleichzeitig hohen Abdeckungsgrad erzielt.

Bei der Ausgestaltung nach Anspruch 29 ist von Vorteil, dass so im Bereich des proximalen Endes der abzudeckenden Nadel die Kanaltiefe in Richtung der Schwenkebene vergrößert wird, wodurch ein zusätzlicher Sammelraum für mögliche aus der Hohlnadel austretende Flüssigkeiten geschaffen wird. Dadurch wird eine noch höhere Sicherheit für das Bedienpersonal geschaffen.

Durch die Weiterbildung nach Anspruch 30 oder 31 wird eine höhere Bediensicherheit für das Schutzelement erzielt, da so die Handhabbarkeit für das Bedienpersonal verbessert wird.

Durch die Ausbildung nach Anspruch 32 wird eine auf die von der Kanalöffnung entgegengesetzt vorragende Bedienfläche am Schutzelement geschaffen, mit welcher ohne händischer Berührung die Verstellung in die Schutzstellung erfolgen kann. Durch Abstützen dieses Abschnitts der Griffleiste an einem festen Gegenstand kann die Verstellung des Schutzelementes in die Schutzstellung erfolgen, ohne dass der Benutzer in diesem Bereich Hand anlegen muss.

Vorteilhaft ist auch eine Ausbildung nach Anspruch 33, da so eine einfache Begrenzung des Kanals erzielt wird.

Von Vorteil sind aber auch Ausbildungen, wie in den Ansprüchen 34 bis 38 beschrieben, da so bei einer möglichen Fehlbedienung ein Durchtreten des proximalen Endes durch den hier den Kanal begrenzenden Wandteil verhindert ist.

Durch die Ausbildung nach Anspruch 39 ist es möglich, einen hohen Überdeckungsgrad des Einstichelementes innerhalb des Schutzelementes zu erzielen, wodurch auch bei unsachgemäßer Bedienung bzw. bei einem nicht vorgesehenen neuerlichen Verwendungsversuch das proximale Ende des Einstichelementes gesichert abgedeckt ist und so Stichverletzung und damit verbundene Infektionen verhindert sind.

Durch die Ausbildung nach Anspruch 40 ist es möglich, einen hohen Überdeckungsgrad des Einstichelementes innerhalb des Schutzelementes zu erzielen, wodurch auch bei unsachgemäßer Bedienung bzw. bei einem nicht vorgesehenen neuerlichen Verwendungsversuch das proximale Ende des Einstichelementes gesichert abgedeckt ist und so Stichverletzung und damit verbundene Infektionen verhindert sind.

Gemäß Anspruch 41 wird eine einfache Formgebungsmöglichkeit zur Ausbildung des Rückhalteelementes geschaffen.

Schließlich wird bei der Ausbildung gemäß Anspruch 42 wird einerseits das Verbringen des Einstichelementes hin zum Kanalgrund erleichtert und andererseits ein Entriegeln, ohne einer damit einhergehender Manipulation, gesichert verhindert.

Die Erfindung wird im nachfolgenden anhand der in den Zeichnungen dargestellten Ausführungsbeispiele näher erläutert.

Es zeigen:
- Fig. 1: eine erfindungsgemäße Baugruppe in schaubildlich vereinfachter Darstellung;
- Fig. 2: die Baugruppe nach Fig. 1 in schaubildlich vereinfachter Darstellung, in voneinander distanzierter Position der einzelnen Bauteile;
- Fig. 3: einen Teilbereich der Handhabungsvorrichtung im Bereich des ersten Halteelements in Seitenansicht geschnitten und vereinfachter, vergrößerter Darstellung;
- Fig. 4: einen Teilbereich der Schutzvorrichtung im Bereich des weiteren Halteelements in Seitenansicht geschnitten und vereinfachter, vergrößerter Darstellung;
- Fig. 5: eine weitere erfindungsgemäße Schutzvorrichtung zur Bildung der Baugruppe in vereinfachter schaubildlicher Darstellung;
- Fig. 6: die Schutzvorrichtung nach Fig. 5 in Ansicht;
- Fig. 7: die Schutzvorrichtung nach den Fig. 5 und 6 in Seitenansicht;
- Fig. 8: die Schutzvorrichtung nach den Fig. 5 bis 7, in Ansicht geschnitten, gemäß den Linien VIII - VIII in Fig. 7;
- Fig. 9: einen Teilbereich der Schutzvorrichtung nach den Fig. 5 bis 8, in Draufsicht geschnitten, gemäß den Linien IX - IX in Fig. 6;
- Fig. 10: einen Teilbereich der Schutzvorrichtung nach den Fig. 5 bis 9, in Unteransicht geschnitten, gemäß den Linien X - X in Fig. 7;
- Fig. 11: die Schutzvorrichtung in deren Schutzstellung nach den Fig. 5 bis 10 in Ansicht geschnitten und vereinfachter Darstellung.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiters können auch Einzelmerkmale oder Merkmalskombinationen aus den gezeigten und beschriebenen unterschiedlichen Ausführungsbeispielen für sich eigenständige, erfinderische oder erfmdungsgemäße Lösungen darstellen.

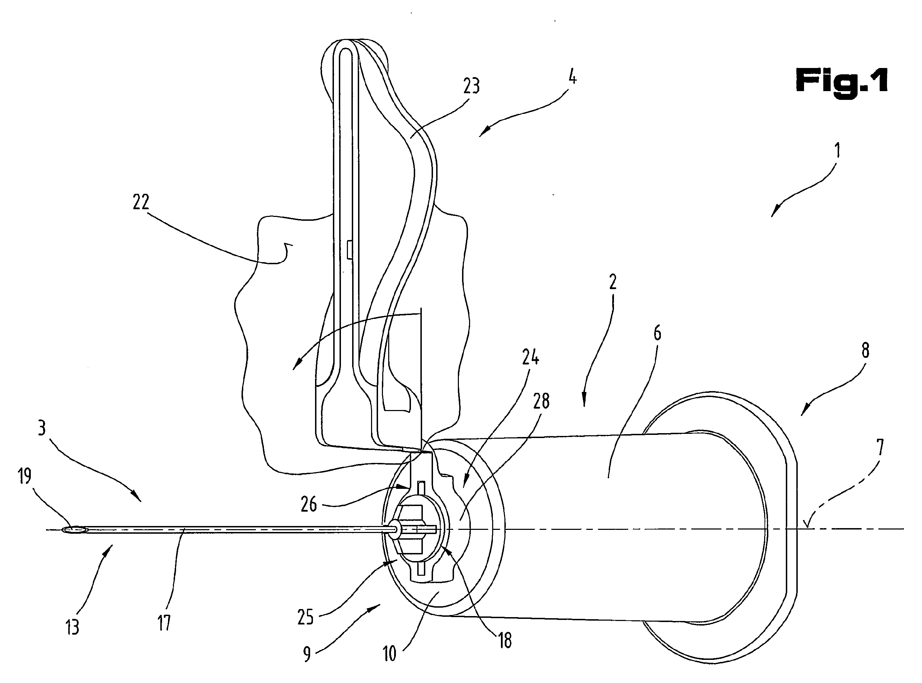

In den Fig. 1 bis 4 ist eine Baugruppe 1, insbesondere für die Medizintechnik, gezeigt, welche zumindest eine Handhabungsvorrichtung 2, ein daran bedarfsweise halterbares Einstichelement 3 sowie eine schwenkbare Schutzvorrichtung 4 für zumindest einen Teilbereich des Einstichelementes 3 umfasst.

Die Handhabungsvorrichtung 2 kann beispielsweise durch einen eine Aufnahmekammer 5 bildenden bzw. umgrenzenden Aufnahmebehälter 6 gebildet sein, der in Richtung einer Längsachse 7 voneinander distanziert eine offene Stirnseite 8 sowie eine zumindest bereichsweise verschlossene Stirnseite 9 aufweist. Bevorzugt wird die zumindest bereichsweise verschlossene Stirnseite 9 durch eine hier vereinfacht dargestellt Stirnwand 10 verschlossen.

Weiters ist im Bereich der zumindest bereichsweise verschlossenen Stirnseite 9 an der Handhabungsvorrichtung 2 noch ein vereinfacht dargestelltes erstes Halteelement 11 sowie ein erstes Kupplungselement 12 bevorzugt im Bereich der Längsachse 7 angeordnet. Das Einstichelement 3 weist bei dem hier gezeigten Ausführungsbeispiel in Richtung der Längsachse 7 voneinander distanziert ein proximales Ende 13 sowie ein distales Ende 14 auf, wobei ein Durchgang 15 die beiden Enden 13, 14 miteinander verbindet. Dadurch ist ein Durchfluss durch das hohl ausgebildete Einstichelement 3 möglich. Allgemein sei hierbei noch hingewiesen, dass die Bezeichnung der beiden Enden 13, 14 auf den Patienten bezogen gewählt wurde. So ist das proximale Ende 13 dem Patienten zugewandt und das distale Ende 14 von diesem abgewandt.

Weiters ist zwischen den beiden Enden 13, 14 ein weiteres Kupplungselement 16 an einer Hohlnadel 17 des Einstichelements 3 feststehend angeordnet. Das erste Kupplungselement 12 am Aufnahmebehälter 6 der Handhabungsvorrichtung 2 bildet mit dem weiteren Kupplungselement 16 an der Hohlnadel 17 des Einstichelements 3 eine Kupplungsvorrichtung 18 zum bedarfsweisen Kuppeln des Einstichelementes 3 an der Handhabungsvorrichtung 2 aus. Dadurch kann das Einstichelement 3, welches auch als Kanüle bezeichnet wird, bedarfsweise lösbar an der Handhabungsvorrichtung 2 gehalten werden.

Ist das Einstichelement 3 - wie hier beim vorliegenden Ausführungsbeispiel gezeigt - als doppelendige Hohlnadel 17 ausgebildet, überragt ein Teilbereich der Hohlnadel 17 - nämlich das proximale Ende 13 - die Handhabungsvorrichtung 2 auf die von der Aufnahmekammer 5 abgewendete Seite. Weiters ist noch im Bereich des proximalen Endes 13 vereinfacht dargestellt, dass in an sich bekannter Weise durch Abschrägung der Hohlnadel 17 eine Öffnung 19 ausgebildet wird, welche durch die schräge Ausrichtung im bezug zur Längsachse 7 annähernd die Form einer Ellipse bzw. ein Ovals ausbildet. Dadurch weist die Öffnung 19 eine längere sowie kürzere Öffnungsachse 20, 21 auf.

Die Schutzvorrichtung 4 weist zumindest ein in einer Schwenkebene 22 verschwenkbar ausgebildetes Schutzelement 23 auf, wobei dieses von einer ersten, den Teilbereich des Einstichelementes 3 freigebenden Stellung (Freigabestellung) in eine zweite, den Teilbereich abdeckenden Stellung (Schutzstellung) verschwenkbar ausgebildet ist.

Zur bedarfsweise lösbaren Halterung bzw. Anbringung weist die Schutzvorrichtung 4 ein weiteres Halteelement 24 auf, mit dem diese feststehend am ersten Halteelement 11 am Aufnahmebehälter 6 der Handhabungsvorrichtung 2 bedarfsweise lösbar gehaltert werden kann. Die beiden Halteelemente 11, 24 bilden eine Haltevorrichtung 25 aus. Die feststehende Halterung der beiden Haltelemente 11, 24 zueinander bedeutet, dass bei vollständigem Eingriff der beiden Halteelemente 11, 24 eine relative Verstellung bzw. Verdrehung der beiden Halteelemente 11, 24 um die Längsachse 7 als auch eine relative axiale Bewegung in Richtung der Längsachse 7 verhindert und somit nicht möglich ist. Dabei ist jedoch aber eine bedarfsweise Anbringung der Schutzvorrichtung 4, insbesondere des Halteelements 24 am ersten Halteelement 11 als auch wiederum eine bedarfsweise Abnahme bzw. Trennung der schwenkbaren Schutzvorrichtung 4 von der Handhabungsvorrichtung 2 möglich. Dabei können die Halteelemente 11, 24 derart zueinander ausgebildet sein, dass eine gegenseitige Halterung durch einen Fest- bzw. Presssitz, eine Konusverbindung oder Rastverbindung realisiert ist.

Zur Erzielung der relativen Lagefixierung bzw. Positionierung der beiden Halteelemente 11, 24 zueinander, ist zwischen diesen beiden Halteelementen 11, 24 der Haltevorrichtung 25 eine Positioniervorrichtung 26 angeordnet, wobei diese bei in Eingriff stehenden Halteelementen 11, 24 die Schwenkebene 22 des Schutzelements 23 der schwenkbaren Schutzvorrichtung 4 in Bezug zur Handhabungsvorrichtung 2 in ihrer Lage festlegt.

Ist das erste Halteelement 11 am Aufnahmebehälter 6 der Handhabungsvorrichtung 2 durch einen etwa rohrförmigen ersten Bauteil 27 gebildet, kann eine Mittelachse dieses Bauteils 27 deckungsgleich zur Längsachse 7 ausgerichtet verlaufend sein. Zumeist der erste rohrförmige Bauteil 27 konzentrisch zur Längsachse 7 am Aufnahmebehälter 6 im Bereich der zumindest bereichsweise verschlossenen Stirnseite 9 an der Stirnwand 10 feststehend angeordnet. Diese mittige bzw. zentrale Anordnung in bezug zum Aufnahmebehälter 6 findet bei einem doppelendig ausgebildeten Einstichelement 3 Anwendung, wobei dann das proximale Ende 13, wie bereits zuvor beschrieben, die Stirnwand 10 auf die von der Aufnahmekammer 5 abgewandte Richtung überragt und das distale Ende 14 bei an der Handhabungsvorrichtung 2 eingesetzter Stellung in die Aufnahmekammer 5 in Richtung auf die offene Stirnseite 8 hin hineinragt. Diese Anordnung des Einstichelements 3 in bezug zur Handhabungsvorrichtung 2 ist in dieser Form allgemein bekannt.

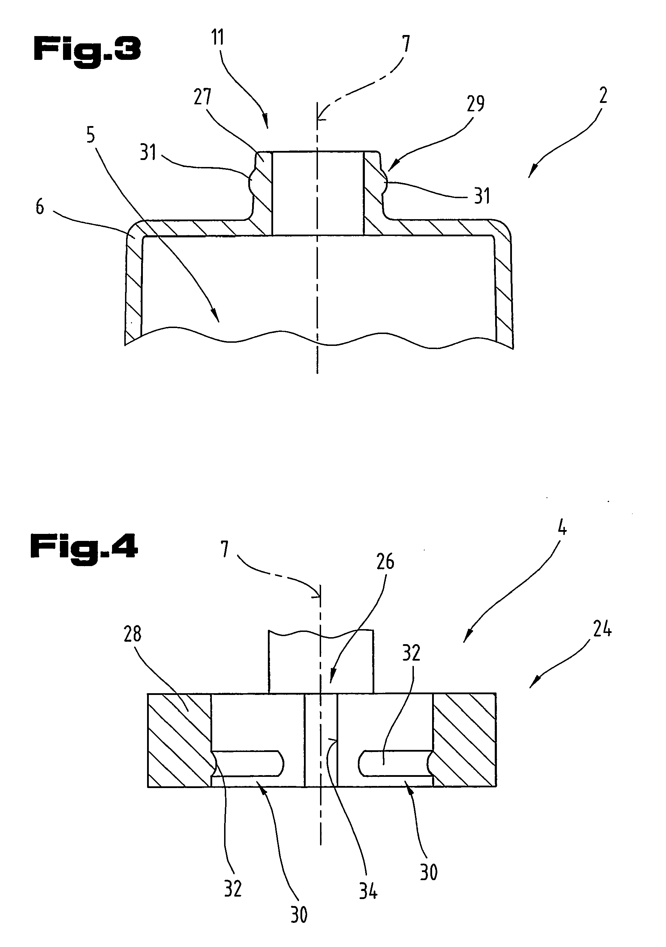

Das erste Halteelement 11 bzw. der dieses bildende rohrförmige Bauteil 27 überragt die Stirnwand 10 auf die von der Aufnahmekammer 5 abgewendete Seite. Das weitere Halteelement 24 der Schutzvorrichtung 4 kann ebenfalls durch einen weiteren, etwa rohrförmig ausgebildeten Bauteil 28 gebildet sein, wobei dieser den ersten rohrförmigen Bauteil 27 im Bereich seiner äußeren Oberfläche, insbesondere im Bereich des Zylindermantels, zumindest bereichsweise übergreift. So entspricht eine äußere Abmessung bzw. ein äußerer Durchmesser des ersten Halteelements 11 annähernd der inneren Abmessung bzw. dem inneren Durchmesser des weiteren Halteelements 24, wodurch aufgrund der gewählten Abmessungen das weitere Halteelement 24 über das erste Halteelement 11 in axialer aufschieb- bzw. aufsetzbar ist. Um ein unbeabsichtigtes Lösen der beiden Halteelemente 11, 24 in der in Eingriff stehenden Stellung zu verhindern, kann die Haltevorrichtung 25 weiters noch miteinander in Eingriff bringbare Rastelemente 29, 30, wie diese am besten aus einer Zusammenschau der Fig. 3 und 4 zu ersehen sind, umfassen. Diese beiden Rastelemente 29, 30 können zumindest bereichsweise über den Umfang der beiden Halteelemente 11, 24 bzw. deren Bauteile 27, 28 an den jeweils einander zugewandten Seiten angeordnet sein.

Selbstverständlich können die zuvor beschriebenen beiden Halteelemente 11, 24 bzw. deren Bauteile 27, 28 aber auch jede andere Raumform wie z.B. oval, mehreckig usw. ausgebildet sein. Durch entsprechende Ausbildung kann aber auch gleichzeitig die Positioniervorrichtung 26 für die orientierte Halterung des Schutzelementes 4 an der Handhabungsvorrichtung 2 gebildet sein.

Bei diesem hier gezeigten Ausführungsbeispiel ist das erste Rastelement 29 - siehe Fig. 3 - am ersten Halteelement 11, nämlich dem hier dargestellten rohrförmigen Bauteil 27 durch einen diesen Bauteil 27 auf die von der Längsachse 7 abgewendete Seite überragenden Wulst 31 gebildet, der zumindest bereichsweise aber auch durchlaufend über den Umfang des Halteelements 11 verlaufend angeordnet sein kann.

Das weitere Rastelement 30 ist am Halteelement 24, beispielsweise dem hier dargestellten rohrförmigen Bauteil 28, auf die der Längsachse 7 zugewendeten Seite überragenden, durch einen weiteren Wulst 32 gebildet, wie dies am besten aus der Fig. 4 ersehen ist. Dieser, das Rastelement 30 bildende Wulst 32 kann wiederum zumindest bereichsweise über den innern Umfang des Halteelements 24, im vorliegenden Fall des rohrförmigen Bauteils 28 verlaufend angeordnet sein, wobei in der eingerasteten Stellung das weitere Rastelement 30 das erste am ersten Halteelement 11 angeordnete Rastelement 29 auf der der Stirnwand 10 zugewendeten Seite übergreift.

Durch diese hier beschriebe, und nur beispielhaft für eine Vielzahl von möglichen anderen Ausbildungen der zusammenwirkende Rastelemente 29, 30 beschriebenen Ausftffirungsform wird erreicht, dass bei in Eingriff stehenden Halteelementen 11, 24 eine unbeabsichtigte Bewegung der beiden Halteelemente 11, 24 in Richtung der Längsachse 7 relativ zueinander verhindert ist. Aufgrund dieser zuvor beschriebenen Ausbildung der zusammenwirkenden Rastelemente 29, 30 ist bei ortsfester Halterung der Handhabungsvorrichtung 2 jedoch eine Schwenkung bzw. Verdrehung der Schutzvorrichtung 4 relativ gegenüber der Handhabungsvorrichtung 2 um die Längsachse 7 möglich. Selbstverständlich kann das Rastelement 29 aber auch z. B. durch eine oder mehrerer nutförmig ausgebildete Vertiefungen am Bauteil 27 und das weitere Rastelement 30 durch einen oder mehrere damit zusammenwirkende bzw. darin eingreifende Vorsprünge am weiteren Bauteil 28 ausgebildet sein.

Wie bereits zuvor beschrieben, ist zwischen den beiden Halteelementen 11, 24 der Haltevorrichtung 25 die Positioniervorrichtung 26 angeordnet, wodurch die Stellung bzw. Position des Schutzelements 23 der Schutzvorrichtung 4 und damit verbunden die Schwenkebene 22 relativ gegenüber der Haridhabungsvorrichtung 2 festgelegt. Die Positioniervorrichtung 26 kann unterschiedlichst ausgebildet sein, wobei im hier gezeigten Ausführungsbeispiel diese im Bereich der Handhabungsvorrichtung 2 durch zumindest ein erstes in Richtung der Längsrichtung 7 sowie senkrecht zu dieser ausgerichtetes Positionierelement 33 und im Bereich des weiteren Halteelements 24 der Schutzvorrichtung 4 durch zumindest ein weiters dazu in etwa gegengleich ausgebildetes Positionierelement 34 gebildet ist.

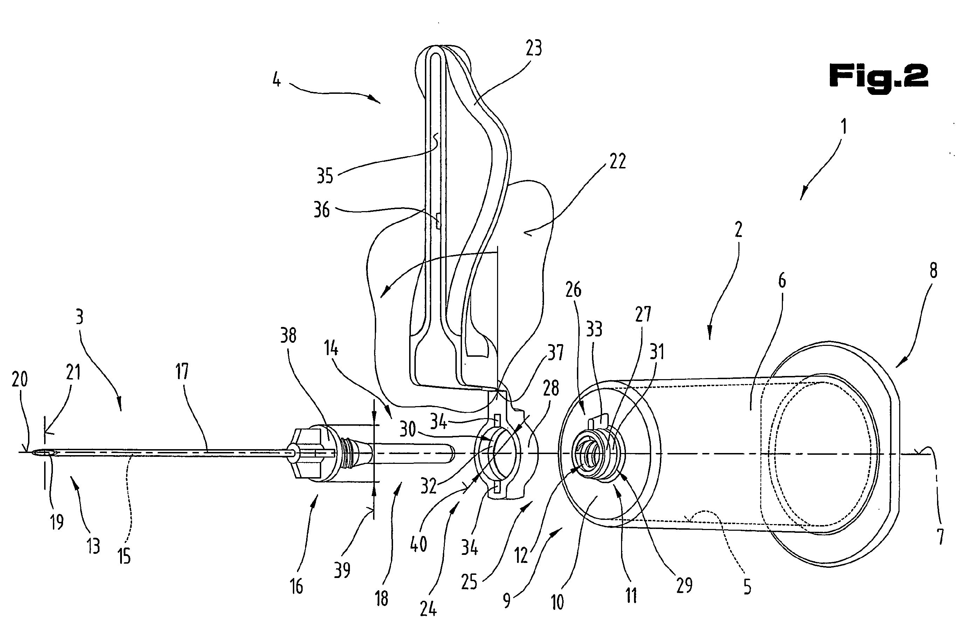

Wie am besten aus der Fig. 2 zu ersehen ist, ist das erste Positionierelement 33 im vorliegenden Fall durch eine Rippe bzw. einen Steg gebildet, der im Bereich der äußeren Oberfläche des ersten Halteelements 11, nämlich dem rohrförmigen Bauteil 27, auf die von der Längsachse 7 abgewendete Seite radial überragend angeordnet ist. Um ein einfaches Aufsetzen der beiden Halteelemente 11, 24 zu ermöglichen, ist das erste Positionierelement 33 in Richtung der Längsachse 7 verlaufend angeordnet. Das weitere Positionierelement 34 im Bereich des weiteren Halteelements 24 kann beispielsweise durch eine gegengleich zum Positionierelement 33 ausgebildete Ausnehmung bzw. einen Schlitz gebildet sein.

Selbstverständlich ist es aber auch möglich, die Positioniervorrichtung 26 am weiteren Halteelement 24 der Schutzvorrichtung 4 durch diametral gegenüberliegend angeordnete Positionierelemente 34, insbesondere wie die zuvor beschriebenen Ausnehmungen bzw. Schlitze, zu bilden. Dadurch kann im Zusammenwirken mit dem ersten Positionierelement 33 an der Handhabungsvorrichtung 2 eine um 180° gedrehte Anbringung der Schutzvorrichtung 4 am Aufnahmebehälter 6 der Handhabungsvorrichtung 2 erzielt werden.

Wie noch vereinfacht aus der Fig. 2 zu ersehen ist, weist das Schutzelement 23 einen Kanal 35 zur Aufnahme zumindest eines Teilbereiches des Einstichelements 3, nämlich der Hohlnadel 17 im Bereich dessen proximalen Endes 13 auf. Um ein unbeabsichtigtes Zurückschwenken des Schutzelements aus der Schutzstellung zu verhindern, ist im Bereich des Kanals 35 mindestens ein Rückhalteelement 36 zur einrastenden Halterung am Teilbereichs des Einstichelementes 3 in der Schutzstellung angeordnet, wie dies bereits aus dem Stand der Technik bekannt ist.

Zur Erzielung der Schwenkbewegung des Schutzelements 23 relativ gegenüber dem weiteren Halteelement 24 kann zwischen diesen Bauteilen ein elastisch verformbarer Steg angeordnet sein. Zur exakteren Festlegung der Schwenkbewegung und der Stellung des Schutzelementes 23 in der dem Teilbereich des Einstichelements 3 abdeckenden Schutzstellung ist es vorteilhaft, wenn das Schutzelement 23 in einer senkrecht zur Schwenkebene 22 ausgerichteten Schwenkachse 37 verschwenkbar ist, wobei diese Schwenkachse 37 bevorzugt durch ein Filmscharnier ausgebildet sein kann.

Durch das Zusammenwirken der in Eingriff stehenden Halteelemente 11, 24 in Verbindung mit der dazwischen angeordneten Positioniervorrichtung 26 sowie gegebenenfalls den zusammenwirlcenden Rastelementen 29, 30 ist die Lage der Schwenkebene 22 relativ zum Aufnahmebehälter 6 der Handhabungsvorrichtung 2 festgelegt. Zumeist ist die Schwenkebene 22 parallel zur Längsachse 7 bzw. in der Längsachse 7 verlaufend ausgerichtet.

Wie bereits zuvor kurz beschrieben, ist zwischen dem Einstichelement 3 und der Handhabungsvorrichtung 2, insbesondere dem Aufnahmebehälter 6, die Kupplungsvorrichtung 18 mit dem ersten am Aufnahmebehälter 6 angeordneten Kupplungselement 12 und dem weiteren, an der Hohlnadel 17 des Einstichelements 3 angeordneten, weiteren Kupplungselement 16 gebildet. Wesentlich dabei ist, dass die beiden Kupplungselemente 12, 16 der Kupplungsvorrichtung 18 zueinander derart ausgebildet bzw. ausgerichtet sind, dass bei vollständig gekuppelter Stellung der beiden Kupplungselemente 12, 16 die kürzere Öffnungsachse 21 der Öffnung 19 etwa parallel zur Schwenkebene 22 oder in der Schwenkebene 22 verlaufend ausgerichtet ist. Betrachtet man nun die durch die Positioniervorrichtung 26 in bezug zum Aufnahmebehälter 6 der Handhabungsvonichtung 2 festgelegte Lage der Schwenkebene 22 ist bei einer einem Benutzer der medizinischen Baugruppe 1 zugewendeten und einsehbaren Öffnung 19 am proximalen Ende 13 das Schutzelement 23 in der Freigabestellung in bezug auf die Handhabungsvorrichtung 2 entweder links oder rechts des abzudeckenden Teilbereiches der Hohlnadel 17 angeordnet. Durch diese gegenseitige Ausrichtung ist bei vollständig gekuppelter Stellung der beiden Kupplungselemente 12, 16 stets ein Blickkontakt zur in etwa elliptisch bzw. oval ausgebildeten Öffnung 19 möglich, wie dies bei der Verwendung der Handhabungsvorrichtung, der Schutzvorrichtung oder der daraus gebildeten medizinischen Baugruppe 1 für das Abnehmen von Körperflüssigkeiten und/oder das Abgeben von fluiden Stoffen in den Körper für medizinische Zwecke notwendig ist. Zumeist wird die Handhabungsvorrichtung 2, die Schutzvorrichtung 4 bzw. die daraus gebildete medizinische Baugruppe 1 auf dem medizinischen Gebiet der Blutabnahme eingesetzt.

In dieser vorliegenden Ausführungsform ist im Bereich der Handhabungsvorrichtung 2 noch dargestellt, dass das erste Kupplungselement 12 und das erste Halteelement 11 zueinander konzentrisch an der zumindest bereichsweise verschlossenen Stirnseite 9 bzw. Stirnwand 10 angeordnet sind. Durch das zuvor beschriebene, rohrförmige Bauteil 27, welche das Halteelement 11 ausbildet, kann die Kupplungsvorrichtung 18 durch eine miteinander in Eingriff stehende Gewindeanordnung gebildet sein, wobei das erste Kupplungselement 12 am rohrförmigen Bauteil 27 durch ein Innengewinde und am weiteren Kupplungselement 16 des Einstichelements 3 durch ein damit in eingriffbares Außengewinde gebildet sein kann. Bevorzugt wird die Gewindeanordnung durch ein doppelgängiges Gewinde gebildet, wodurch eine Einsetzmöglichkeit und eine damit verbundene Endposition der Öffnung 19 am Einstichelement 3 jeweils um 180 ° geschwenkt zur Schwenkebene 22 erzielbar ist. Durch die zuvor beschriebene Anordnung und Ausbildung der Kupplungselemente 12, 16 zueinander wird dann stets erreicht, dass die kürzere Öffnungsachse 21 der Öffnung 19 entweder von der vorderen bzw. hinteren Betrachtungsseite der Schwenkebene 22 einzusehen ist.

Weiters ist damit sichergestellt, dass das Schutzelement 23 nicht unter einem Winkel in bezug zur Öffnungsachse 21 verschwenkbar ist und so beispielsweise bei einer Blutabnahme das Schutzelement 23 bei der bestimmungsgemäßen Verwendung nicht dem Arm des Patienten zugewendet ist und somit einer Abnahme hinderlich ist. Bei einer dazu in etwa um 180 ° gedrehten Stellung des Schutzelements 23 verhindert dieses einen Einblick bzw. die Betrachtung der Öffnung 19, welche für die Durchführung des Einstichvorganges in einen Körperteil, wie beispielsweise einer Vene oder einer Ader, notwendig ist, um einen bestimmungsgemäßen Einstichvorgang durchführen zu können.

Anstelle der Gewindeanordnung zur Bildung der Kupplungselemente 12, 16 kann aber auch die Kupplungsvorrichtung 18 beispielsweise durch eine Bajonett - Anordnung oder dgl. gebildet sein. Gleichfalls kann aber auch die Kupplungsvorrichtung 18 durch eine Konusverbindung realisiert sein, bei welcher wiederum in der gekuppelten Stellung sichergestellt sein muss, dass die kürzere Öffnungsachse 21 der Öffnung 19 etwa parallel zur Schwenkebene 22 oder in der Schwenkebene verlaufend ausgerichtet ist. Dies kann durch eine zusätzliche Positioniervorrichtung zwischen den in Eingriff bringbaren Teilen erreicht werden.

Vorteilhaft ist bei dieser medizinischen Baugruppe 1, dass die Schutzvorrichtung 4 unabhängig vom Einstichelement 3 bereits an der Handhabungsvorrichtung vormontierbar ist und erst bei bestimmungsgemäßer Verwendung das Einstichelement 3 an der Handhabungsvorrichtung 2 anzubringen ist - im vorliegenden Fall eingeschraubt werden kann. Während des bestimmungsgemäßen Gebrauches ist das Schutzelement 23 seitlich der Handhabungsvorrichtung 2 in der Schwenkebene 22 angeordnet, ohne dass dabei eine hinderliche Stellung entweder zwischen dem Arm des Patienten und der Handhabungsvorrichtung 2 oder ein Blickkontakt zu der dem Anwender, beispielsweise einem Arzt, zugewendeten Öffnung 19 des Einstichelements 3 behindert wird.

Um einen besseren Festsitz der beiden zusammenwirkenden Halteelemente 11, 24 zu erzielen oder aber auch ein unbeabsichtigtes Lösen dieser beiden voneinander zu verhindern, kann es vorteilhaft sein, wenn ein der Stirnwand 10 des Aufnahmebehälters 6 zugewendeter Anschlagteil 38 des weiteren Kupplungselements 16 in einer senkrecht zur Längsachse 7 ausgerichteten Ebene eine größere Abmessung 39 gegenüber einer inneren Abmessung 40 des weiteren Halteelements 24 aufweist. Dadurch übergreift das Anschlagteil 38 zumindest einen Teilbereich des rohrförmig ausgebildeten Bauteils 28 und verhindert so ein unbeabsichtigtes Abnehmen der Schutzvorrichtung 4 von der Handhabungsvorrichtung 2.

Die hier beschriebene Handhabungsvorrichtung 2, die Schutzvorrichtung 4 sowie das Kupplungselement 16 am Einstichelement 3 werden zumeist aus einem Kunststoff hergestellt, wobei die Handhabungsvorrichtung 2 bevorzugt transparent bzw. durchsichtig ausgebildet ist, um einen Einblick in den Innenraum zu ermöglichen. Wesentlich ist bei dieser Baugruppe bzw. der diese bildenden Bauteile, dass das Einstichelement 3, z.B. die Kanüle mit ihrer Öffnung 19 orientiert in bezug zum Kupplungselement 16 und die Schutzvorrichtung 4 orientiert bzw. in ihrer Position an der Handhabungsvorrichtung 2 in bezug zum Kupplungselement 12 festgelegt ist.

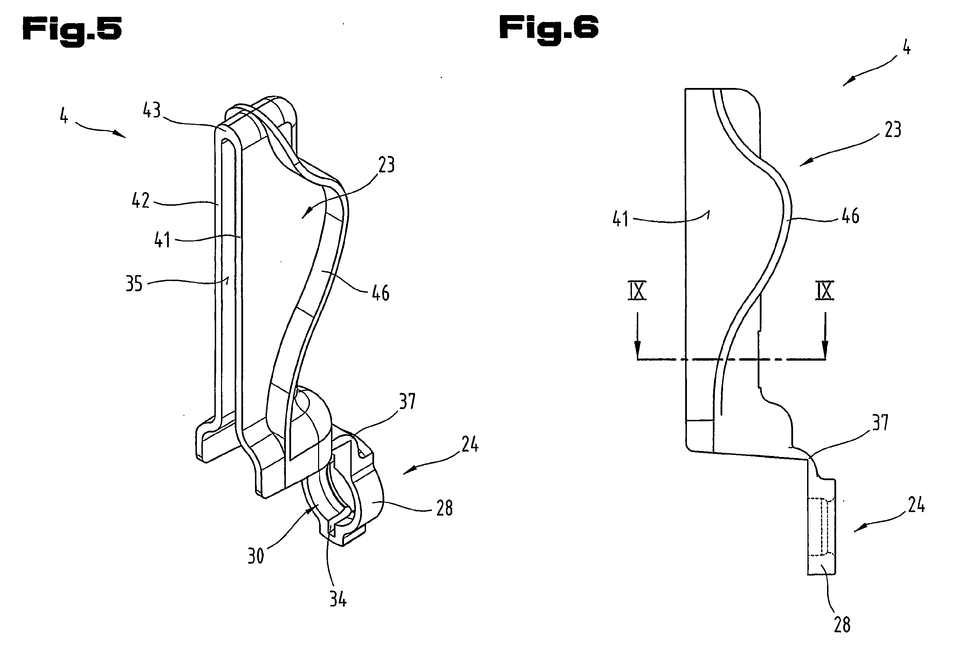

In den Fig. 5 bis 11 ist eine weitere und gegebenenfalls für sich unabhängige Ausführungsform der Schutzvorrichtung 4 zur Bildung der medizinischen Baugruppe 1 dargestellt, wobei wiederum für gleiche Teile gleiche Bezugszeichen bzw. Bauteilbezeichnungen, wie in den vorangegangenen Fig. 1 bis 4 verwendet werden. Gleichfalls wird, um unnötige Wiederholungen zu vermeiden, auf die detaillierte Beschreibung in den vorangegangenen Fig. 1 bis 4 hingewiesen bzw. Bezug genommen.

Auch diese Schutzvorrichtung 4 dient wiederum zur bedarfsweisen Abdeckung des hier teilweise dargestellten Einstichelementes 3 im Zusammenwirken mit der Handhabungsvorrichtung 2. So weist diese Schutzvorrichtung 4 wiederum das Schutzelement 23, das weitere Halteelement 24 zum Haltern bzw. Kuppeln mit dem ersten Halteelement 11 an der Handhabungsvorrichtung 2 mittels der diese bildende Haltevorrichtung 25 auf.

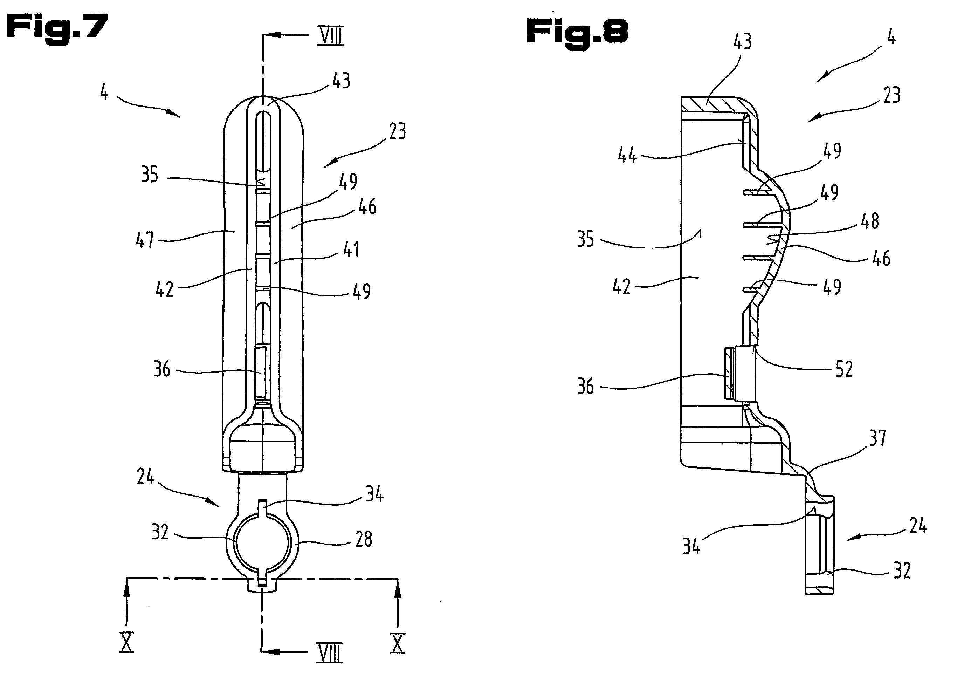

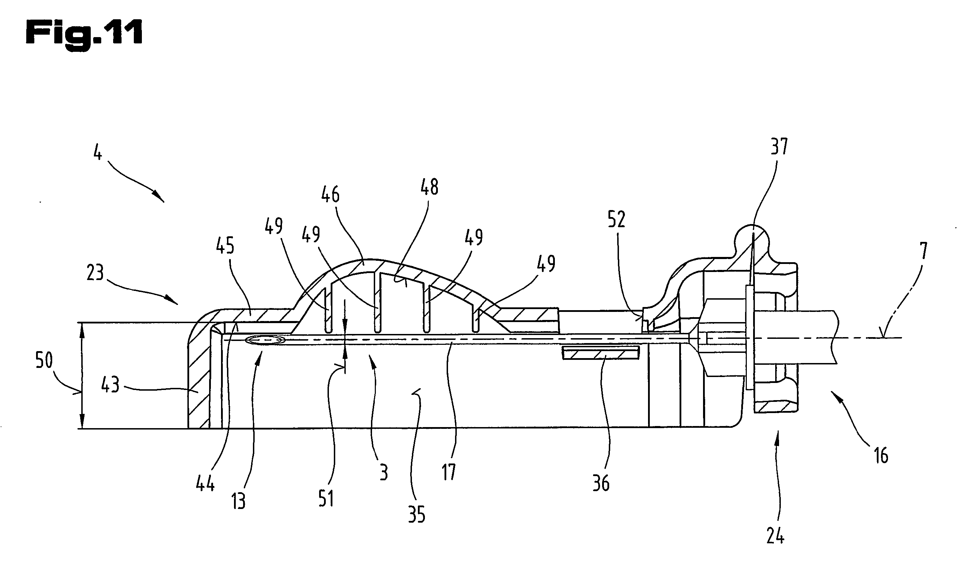

Das Halteelement 24 weist auch das bereits zuvor detailliert beschriebene Rastelement 30 sowie Positionierelement 34 auf. Das Schutzelement 23 ist wiederum über die Schwenkachse 37 in der Schwenkebene 22 ausgehend von der den Teilbereich des Einstichelement 3 freigebenden Stellung (Freigabestellung) in die zweite, den Teilbereich abdeckenden Stellung (Schutzstellung) verschwenkbar. Der Kanal 35 ist bei diesem Ausführungsbeispiel über seine Längserstreckung durch parallel zueinander verlaufende und senkrecht zur Längserstreckung voneinander distanzierte Stege 41, 42 begrenzt. Weiters ist der Kanal 35 auf der vom weiteren-Halteelement 24 abgewendeten Seite mit einer Stirnwand 43 begrenzt, welche die beiden Stege auf der vom weiteren Halteelement abgewendeten Seite miteinander verbindet. Weiters ist aus einer Zusammenschau der Fig. 5 und 7 zu ersehen, dass die beiden Stege 41, 42 im Nahbereich des weiteren Halteelementes 24 weiter voneinander distanziert sind, als in ihrer weiteren Längserstreckung. Diese Verbreiterung des Kanals 35 dient dazu, um einen Teil des weiteren Kupplungselementes 16 - siehe Darstellung in Fig. 11- schützend im Kanal 35 aufzunehmen und gleichzeitig abzudecken.

Wie am Besten aus einer Zusammenschau der Fig. 8 und 11 zu ersehen ist, ist im Kanal 35 5 wiederum zumindest ein Rückhalteelement 36 angeordnet, welches bei diesem Ausführungsbeispiel in etwa in einer ersten Hälfte der Längserstreckung des Kanals 35, ausgehend vom weiteren Halteelement 24 angeordnet ist. Bevorzugt ist das Rückhalteelement 36 in etwa im Drittel der gesamten Längserstreckung des Kanals 35, ausgehend vom weiteren Halteelement 24 in diesem vorgesehen. Es ist aber auch möglich, dass das Rückhalteelement 36 in der abdeckenden Stellung (Schutzstellung) des Schutzelementes 23 im Abschnitt zwischen dem weiteren Halteelement 24 der Schutzvorrichtung 4 und einer halben Distanz zwischen dem proximalen Ende 13 und dem weiteren Halteelement 24 angeordnet ist.

Zur besseren Einrastung der Schutzvorrichtung 4, insbesondere des Schutzelementes 23 am abzudeckenden Teil des Einstichelement 3, weist das Rückhalteelement 36 eine Längserstreckung in Richtung des Kanals 35 zwischen 3 mm und 10 mm, bevorzugt zwischen 5 mm und 7 mm, auf. Diese größere Längserstreckung des Rückhalteelementes 36 dient dazu, um in der verrasteten Stellung des Schutzelementes 23 an dem Einstichelement 3 eine bessere Führung und Lagefixierung zu erzielen.

Im Bereich eines Kanalgrundes 44 weist das Schutzelement 23 einen die beiden Stege 41, 42 zumindest bereichsweise verbindenden Wandteil 45 auf, wodurch der Kanal 35 nunmehr auf zumindest vier Seiten das abzudeckende Einstichelement 3 in der abdeckenden Stellung (Schutzstellung) abdeckt bzw. umgeben ist.

Weiters ist es vorteilhaft, wenn zumindest einer der beiden Stege 41, 42 in senkrechter Richtung zu diesem auf der vom Kanal 35 abgewendeten Seite durch eine Griffleiste 46, 47 überragt ist. Bevorzugt sind die Griffleisten 46, 47 symmetrisch zu den Stegen 41, 42 angeordnet und überragen beide Stege 41, 42 auf die vom Kanal 35 abgewendete Seite in senkrechter Richtung zu diesen. Zur besseren Handhabung können die Griffleisten 46, 47 jeweils über ihre Längserstreckung gesehen einen geschwungenen Längsverlauf aufweisen, wie dies am Besten aus den Fig. 5 und 6 zu ersehen ist.

Weiters ist aus den Darstellungen der Fig. 8 und 11 zu ersehen, dass der Kanal 35 zwischen dem Rückhalteelement 36 und der Stirnwand 43 eine zusätzliche konkave Vertiefung 48 aufweist und so der Kanal 35 in diesem Bereich einen vertieften Kanalgrund 44 aufweist. Diese zusätzliche konkave Vertiefung 48 dient dazu, dass einerseits im Bereich der äußeren Oberfläche die Handhabbarkeit des Schutzelementes 23 verbessert und andererseits ein Aufnahmeraum für mögliche aus dem Einstichelement 3 austretende Flüssigkeiten geschaffen wird.

Im Bereich der konkaven Vertiefung 48 des Kanals 35 begrenzt bei diesem Ausführungsbeispiel eine der Griffleisten 46, 47 den Kanal 35 im Bereich seines vertieften Kanalgrundes 44. Zur Erzielung einer besseren Abstützung und Führung des im Kanal 35 aufzunehmenden Teils des Einstichelementes 3 ist im Bereich der konkaven Vertiefung 48 mindestens ein Stützsteg 49 angeordnet, wobei sich der oder die Stützstege 49 zwischen den beiden Stegen 41, 42 erstrecken und diese miteinander verbinden. Dabei sind der oder die Stützstege 49 in senkrechter Richtung zu den Stegen 41, 42 sowie gegebenenfalls in senkrechter Richtung zur Längserstreckung des Kanals 35 ausgerichtet. Weiters enden der oder die Stützstege 49 ausgehend von der konkaven Vertiefung 48 im Bereich des Kanalgrundes 44 des Kanals 35.

Wie nun weiters am Besten aus der Fig. 11 zu entnehmen ist, verläuft der Kanalgrund 44 in der abdeckenden Stellung (Schutzstellung) unmittelbar benachbart zu der durch das weitere Halteelement 24 verlaufenden Längsachse 7, wodurch der abzudeckende Teil des Einstichelements 3 unmittelbar benachbart dem Kanalgrund 44 angeordnet ist. Um bei unsachgemäßer Behandlung des Schutzelementes 23 einen Austritt des proximalen Endes 13 des Einstichelements 3 aus dem Kanal 35 zu vermeiden, beträgt eine Kanaltiefe 50 des Kanals 35 in senkrechter Richtung zur Längserstreckung desselben ein Vielfaches, wie beispielsweise in 5- bis 20faches einer äußeren Abmessung 51 des abzudeckenden Teils des Einstichelementes 3. Dadurch ist gewährleistet, dass auch bei einem Versuch das Schutzelement 23 entgegen der Schließbewegung von der eingerasteten Stellung vom Einstichelement 3 wegzubewegen, auf alle Fälle das proximale Ende 13 und damit die eine hohe Verletzungsgefahr aufweisende Spitze sicher abgedeckt ist. Die Kanaltiefe 50 ist stark von der äußeren Abmessung 51 abhängig und kann auch ein 10- bis 15-faches davon betragen.

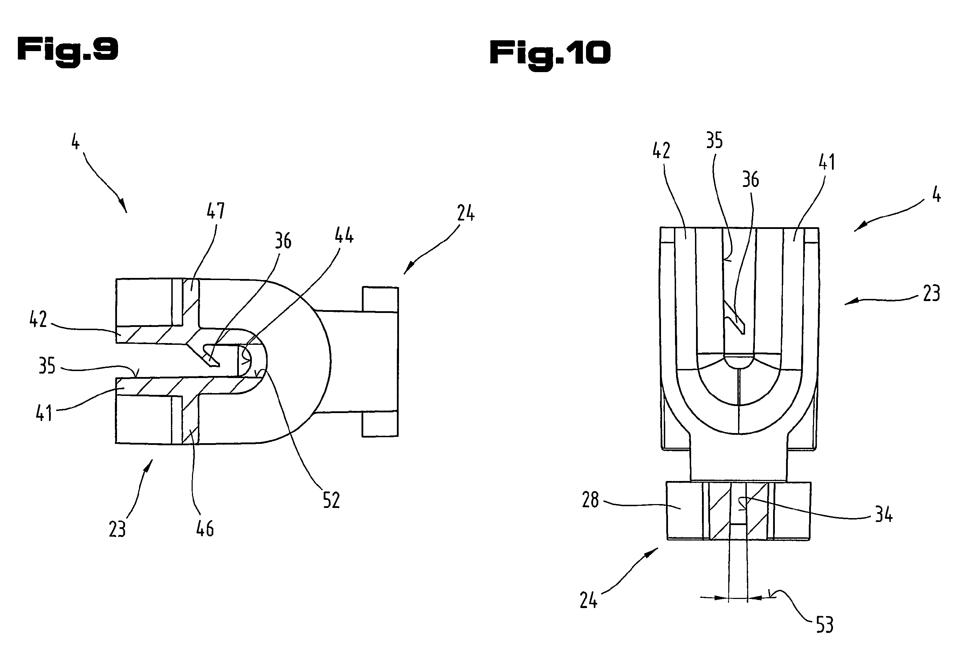

Zur besseren Ausformung des Rückhalteelementes 36 ist es vorteilhaft, wenn der den Kanalgrund 44 begrenzende Wandteil 45 des Kanals 35 im Bereich des Rückhalteelementes 36 eine Öffnung 52 aufweist. Das Rückhalteelement 36 ist, wie dies am Besten aus den Fig. 9 und 10 zu ersehen ist, auf einem der Stege, im vorliegenden Ausführungsbeispiel am Steg 42, innerhalb des Kanals 35 angeordnet und ausgehend von diesem in Richtung auf den Kanalgrund 44 in den Kanal 35 vorragend ausgebildet. Dabei weist das Rückhalteelement 36 durch die winkelig verlaufende Stellung und seine gewählte Wandstärke gewisse elastische Verformungseigenschaften auf, um einen Durchtritt des Einstichelementes 3 zwischen dem Ende des Halteelementes 36 und dem diesen gegenüberliegenden Steg 41 hin zum Kanalgrund 44 zu ermöglichen. Um eine Entriegelung des Schutzelementes 23 von der eingerasteten Stellung zu vermeiden, weist das Rückhalteelement 36 in die entgegengesetzte Betätigungsrichtung eine ausreichende Steifigkeit auf, wobei bei der erneuten Öffnungsbewegung die Distanz zwischen dem Ende des Rückhalteelementes 36 und dem gegenüberliegenden Steg 41 stets verringert wird.

Das Schutzelement 23 ist, wie bereits zuvor beschrieben, mittels dem als Schwenkachse 37 ausgebildeten Firmscharnier mit dem weiteren Halteelement 24 schwenkbar verbunden, wobei das Filmscharnier vor der ersten Verstellung in die abdeckende Stellung (Schutzstellung) eine überwindbare Lagefixierung durch die dem Material innewohnenden Eigenschaften für das Schutzelement bildet.

Schließlich ist aus der Darstellung der Fig. 10 noch zu ersehen, dass das hier spalt- bzw. schlitzförmig ausgebildete Positionierelement 34 ausgehend von dem der Handhabungsvorrichtung 2 zuwendbaren Teil des Halteelementes 24 stetig um einen Winkel 53 verengende Begrenzungswände aufweist, wodurch beim Aufsetzen des Halteelementes 24 auf das erste Halteelement 11 der Handhabungsvorrichtung 2 eine bessere Lagefixierung mit dem dort angeordneten Positionierelement 33, welcher steg- bzw. rippenförmig ausgebildet ist, erzielbar ist.

Es ist aber unabhängig dazu noch möglich, die zuvor beschriebene Baugruppe nicht nur in der Medizintechnik - hier für die Blutabnahme, das Aufsammeln von anderen Körperflüssigkeiten (Urin und andere Ausscheidungsstoffe) - sondern auch für das Aufsammeln bzw. Aufnehmen von Proben in den verschiedensten Anwendungsbereichen - wie z.B. Gefahrenstoffe, Chemische Produkte usw. - einzusetzen.

Der Ordnung halber sei abschließend darauf hingewiesen, dass zum besseren Verständnis des Aufbaus der medizinischen Baugruppe diese bzw. deren Bestandteile teilweise unmaßstäblich und/oder vergrößert und/oder verkleinert dargestellt wurden.

Vor allem können die einzelnen in den Fig. 1, 2; 3; 4; 5 bis 11 gezeigten Ausführungen den Gegenstand von eigenständigen , erfindungsgemäßen Lösungen bilden. Die den eigenständigen erfinderischen Lösungen zugrundeliegende Aufgabe kann der Beschreibung entnommen werden.

### Bezugszeichenaufstellung

- 1: Baugruppe
- 2: Handhabungsvorrichtung
- 3: Einstichelement
- 4: Schutzvorrichtung
- 5: Aufnahmekammer

- 6: Aufnahmebehälter
- 7: Längsachse
- 8: Stirnseite
- 9: Stirnseite
- 10: Stirnwand

- 11: Halteelement
- 12: Kupplungselement
- 13: proximales Ende
- 14: distales Ende
- 15: Durchgang

- 16: Kupplungselement
- 17: Hohlnadel
- 18: Kupplungsvorrichtung
- 19: Öffnung
- 20: Öffnungsachse

- 21.: Öffnungsachse
- 22: Schwenkebene
- 23: Schutzelement
- 24: Haltelement
- 25: Haltevorrichtung

- 26: Positioniervorrichtung
- 27: Bauteil
- 28: Bauteil
- 29: Rastelement
- 30: Rastelement

- 31: Wulst
- 32: Wulst
- 33: Positionierelement
- 34: Positioniereleinent
- 35: Kanal

- 36: Rückhalteelement
- 37: Schwenkachse
- 38: Anschlagteil
- 39: Abmessung
- 40: Abmessung

- 41: Steg
- 42: Steg
- 43: Stirnwand
- 44: Kanalgrund
- 45: Wandteil.

- 46: Griffleiste
- 47: Griffleiste
- 48: Vertiefung
- 49: Stützsteg
- 50: Kanaltiefe

- 51: äußere Abmessung
- 52: Öffnung
- 53: Winkel

## Patentansprüche

1. Baugruppe (1), insbesondere für die Medizintechnik, umfassend eine Handhabungsvorrichtung (2) mit einem daran angeordneten ersten Halteelement (11) sowie einem ersten Kupplungselement (12), ein Einstichelement (3) mit einem proximalen sowie einem distalen Ende (13, 14), die in Richtung einer Längsachse (7) voneinander distanziert sind, wobei ein Durchgang (15) die beiden Enden (13, 14) miteinander verbindet, und einem zwischen den Enden (13, 14) angeordneten weiteren Kupplungselement (16) , wobei die beiden Kupplungselemente (12, 16) eine Kupplungsvorrichtung (18) ausbilden und das Einstichelement (3) an der Handhabungsvorrichtung (2) bedarfsweise lösbar gehalten ist, wobei das proximale Ende (13) des Einstichelements (3) die Handhabungsvorrichtung (2) überragt und zumindest am proximalen Ende (13) eine Öffnung (19) mit einer längeren und kürzeren Öffnungsachse (20, 21) angeordnet ist, eine schwenkbare Schutzvorrichtung (4) für zumindest einen Teilbereich des Einstichelementes (3), und die Schutzvorrichtung (4) bedarfsweise lösbar mit einem weiteren Halteelement (24) feststehend am ersten Halteelement (11) der Handhabungsvorrichtung (2) gehalten ist, wobei die beiden Halteelemente (11, 24) eine Haltevorrichtung (25) ausbilden und ein Schutzelement (23) der Schutzvorrichtung (4) in einer in der Längsachse (7) verlaufend ausgerichteten Schwenkebene (22) von einer ersten, den Teilbereich des Einstichelements (3) freigebenden Stellung (Freigabestellung) in eine zweite, den Teilbereich abdeckenden Stellung (Schutzstellung) verschwenkbar ist, wobei zwischen den beiden Halteelementen (11, 24) der Haltevorrichtung (25) eine Positioniervorrichtung (26) angeordnet ist und diese bei in Eingriff stehenden Halteelementen (11, 24) die Schwenkebene (22) des Schutzelementes (23) der Schutzvorrichtung (4) in bezug zur Handhabungsvorrichtung (2) in ihrer Lage festlegt und dass die beiden Kupplungselemente (12, 16) der Kupplungsvorrichtung (18) zueinander derart ausgebildet sind, dass bei vollständig gekuppelter Stellung der beiden Kupplungselemente (12, 16) die kürzere Öffnungsachse (21) der Öffnung (19) etwa parallel zur Schwenkebene (22) oder in der Schwenkebene (22) verlaufend ausgerichtet ist, **dadurch gekennzeichnet, dass** die Positioniervorrichtung (26) im Bereich der Handhabungsvorrichtung (2) durch zumindest ein erstes in Richtung der Längsachse (7) sowie senkrecht zu dieser ausgerichtetes Positionierelement (33) und im Bereich des weiteren Halteelementes (24) durch zumindest ein weiteres dazu in etwa gegengleich ausgebildetes Positionierelement (34) gebildet ist.

2. Baugruppe (1) nach Anspruch 1, **dadurch gekennzeichnet, dass** die Handhabungsvorrichtung (2) durch zumindest einen eine Aufnahmekammer (5) bildenden Aufnahmebehälter (6) mit einer zumindest bereichsweise von einer Stirnwand (10) verschlossenen Stirnseite (8, 9) gebildet ist.

3. Baugruppe (1) nach Anspruch 1 oder 2, **dadurch gekennzeichnet, dass** das erste Kupplungselement (12) und das erste Halteelement (11) zueinander konzentrisch an der zumindest bereichsweise verschlossenen Stirnseite (9) angeordnet sind.

4. Baugruppe (1) nach einem der vorhergehenden Ansprüche, **dadurch gekennzeichnet, dass** das erste Halteelement (11) durch einen in etwa rohrförmigen ersten Bauteil (27) gebildet ist.

5. Baugruppe (1) nach Anspruch 4, **dadurch gekennzeichnet, dass** der rohrförmigen Bauteil (27) die Stirnwand (10) auf die von der Aufnahmekammer (5) abgewendete Seite überragt.

6. Baugruppe (1) nach einem der vorhergehenden Ansprüche, **dadurch gekennzeichnet, dass** das weitere Halteelement (24) durch einen weiteren etwa rohrförmigen Bauteil (28) gebildet ist und dieser den ersten rohrförmigen Bauteil (27) an seiner äußeren Oberfläche zumindest bereichsweise übergreift.

7. Baugruppe (1) nach einem der vorhergehenden Ansprüche, **dadurch gekennzeichnet, dass** die Haltevorrichtung (25) weiters noch miteinander in Eingriff bringbare Rastelemente (29, 30) umfasst, die zumindest bereichsweise an den beiden Halteelementen (11, 24) angeordnet sind.

8. Baugruppe (1) nach Anspruch 7, **dadurch gekennzeichnet, dass** das erste Rastelement (29) am ersten Halteelement (11) durch einen den rohrförmigen Bauteil (27) auf die von einer Längsachse (7) abgewendete Seite überragenden Wulst (31) gebildet und zumindest bereichsweise über den Umfang verlaufend angeordnet ist.

9. Baugruppe (1) nach Anspruch 7 oder 8, **dadurch gekennzeichnet, dass** das weitere Rastelement (30) am weiteren Halteelement (24) durch einen weiteren den rohrförmigen Bauteil (28) auf die der Längsachse (7) zugewendeten Seite überragenden Wulst (32) gebildet und zumindest bereichsweise über den Umfang verlaufend angeordnet ist und in der eingerasteten Stellung das weitere Rastelement (30) das erste Rastelement (29) auf der der Stirnwand (10) zugewendeten Seite übergreift.

10. Baugruppe (1) nach einem der vorhergehenden Ansprüche, **dadurch gekennzeichnet, dass** das Schutzelement (23) um eine senkrecht zur Schwenkebene (22) ausgerichtete Schwenkachse (37) verschwenkbar ist.

11. Baugruppe (1) nach Anspruch 10, **dadurch gekennzeichnet, dass** die Schwenkachse (37) durch ein Filmscharnier gebildet ist.

12. Baugruppe (1) nach Anspruch 11, **dadurch gekennzeichnet, dass** das Filmscharnier vor der ersten Verstellung in die abdeckende Stellung (Schutzstellung) eine überwindbare Lagefixierung für das Schutzelement (23) bildet.

13. Baugruppe (1) nach einem der vorhergehenden Ansprüche, **dadurch gekennzeichnet, dass** die Kupplungsvorrichtung (18) durch eine miteinander in Eingriff stehende Gewindeanordnung gebildet ist.

14. Baugruppe (1) nach Anspruch 13, **dadurch gekennzeichnet, dass** die Gewindeanordnung durch ein doppelgängiges Gewinde gebildet ist.

15. Baugruppe (1) nach einem der vorhergehenden Ansprüche, **dadurch gekennzeichnet, dass** ein der Stirnwand (10) zugewendeter Anschlagteil (38) des weiteren Kupplungselementes (16) in einer senkrecht zur Längsachse (7) ausgerichteten Ebene eine größere Abmessung (39) gegenüber einer innere Abmessung (40) des weiteren Halteelementes (24) aufweist.

16. Baugruppe (1) nach Anspruch 1, **dadurch gekennzeichnet, dass** das erste Positionierelement (33) durch eine Rippe oder einen Steg gebildet ist.

17. Baugruppe (1) nach Anspruch 1 oder 16, **dadurch gekennzeichnet, dass** das erste Positionierelement (33) am rohrförmigen Bauteil (27) des ersten Halteelementes (11) angeordnet ist und dieses auf die von der Längsachse (7) abgewendete Seite radial überragt.

18. Baugruppe (1) nach Anspruch 1, **dadurch gekennzeichnet, dass** das weitere Positionierelement (34) durch eine Ausnehmung oder einen Schlitz gebildet ist.

19. Baugruppe (1) nach einem der vorhergehenden Ansprüche, **dadurch gekennzeichnet, dass** das Schutzelement (23) der Schutzvorrichtung (4) in der das Einstichelement (3) zumindest bereichsweise abdeckenden Stellung eingerastet am Einstichelement (3) gehalten ist.

20. Baugruppe (1) nach einem der vorhergehenden Ansprüche, **dadurch gekennzeichnet, dass** das Schutzelement (23) einen Kanal (35) zur Aufnahme zumindest einen Teilbereiches des Einstichelementes (3) aufweist.

21. Baugruppe (1) nach Anspruch 20, **dadurch gekennzeichnet, dass** der Kanal (35) auf zumindest vier Seiten das abzudeckende Einstichelement (3) in der abdeckenden Stellung (Schutzstellung) abdeckt.

22. Baugruppe (1) nach einem der Ansprüche 19 bis 21, **dadurch gekennzeichnet, dass** im Kanal (35) mindestens ein Rückhalteelement (36) zur einrastenden Halterung des Teilbereiches des Einstichelementes (3) in der Schutzstellung angeordnet ist.

23. Baugruppe (1) nach einem der Ansprüche 19 bis 22, **dadurch gekennzeichnet, dass** das Rückhalteelement (36) in der abdeckenden Stellung (Schutzstellung) des Schutzelements (23) im Abschnitt zwischen dem weiteren Halteelement (24) der Schutzvorrichtung (4) und einer halben Distanz zwischen dem proximalen Ende (13) und dem weiteren Halteelement (24) angeordnet ist.

24. Baugruppe (1) nach Anspruch 23, **dadurch gekennzeichnet, dass** das Rückhalteelement (36) in etwa im Drittel der gesamten Längserstreckung des Kanals (35) ausgehend vom weiteren Halteelement (24) angeordnet ist.

25. Baugruppe (1) nach einem der Ansprüche 20 bis 24, **dadurch gekennzeichnet, dass** der Kanal (35) auf der vom weiteren Halteelement (24) abgewendeten Seite mit einer Stirnwand (43) begrenzt ist.

26. Baugruppe (1) nach einem der Ansprüche 20 bis 25, **dadurch gekennzeichnet, dass** der Kanal (35) über seine Längserstreckung durch parallel zueinander verlaufende und senkrecht zur Längserstreckung voneinander distanzierte Stege (41, 42) begrenzt ist.

27. Baugruppe (1) nach Anspruch 26, **dadurch gekennzeichnet, dass** die Stege (41, 42) im Nahbereich des weiteren Halteelementes (24) weiter voneinander distanziert sind als in ihrer weiteren Längserstreckung.

28. Baugruppe (1) nach Anspruch 26 oder 27, **dadurch gekennzeichnet, dass** die beiden Stege (41, 42) auf der vom weiteren Halteelement (24) abgewendeten Seite über die Stirnwand (43) miteinander verbunden sind.

29. Baugruppe (1) nach einem der Ansprüche 20 bis 28, **dadurch gekennzeichnet, dass** der Kanal (35) zwischen dem Rückhalteelement (36) und der Stirnwand (43) eine zusätzliche konkave Vertiefung (48) aufweist.

30. Baugruppe (1) nach einem der Ansprüche 26 bis 29, **dadurch gekennzeichnet, dass** zumindest einer der Stege (41, 42) in senkrechter Richtung zu diesem auf der von Kanal (35) abgewendeten Seite durch eine Griffleiste (46, 47) überragt ist.

31. Baugruppe (1) nach Anspruch 30, **dadurch gekennzeichnet, dass** die Griffleisten (46, 47) die beiden Stege (41, 42) auf die vom Kanal (35) abgewendete Seite überragen.

32. Baugruppe (1) nach Anspruch 30 oder 31, **dadurch gekennzeichnet, dass** die Griffleiste (46, 47) über ihre Längserstreckung einen geschwungenen Längsverlauf aufweist.

33. Baugruppe (1) nach einem der Ansprüche 30 bis 32, **dadurch gekennzeichnet, dass** die Griffleiste (45, 46) den Kanal (35) im Bereich der konkaver Vertiefung (48) begrenzt.

34. Baugruppe (1) nach Anspruch 29 oder 33, **dadurch gekennzeichnet, dass** im Kanal (35) im Bereich der konkaver Vertiefung (48) mindestens ein Stützsteg (49) zur Abstützung und Führung des im Kanal (35) aufzunehmenden Teils des Einstichelements (3) angeordnet ist.

35. Baugruppe (1) nach Anspruch 34, **dadurch gekennzeichnet, dass** sich der oder die Stützstege (49) zwischen den beiden Stegen (41, 42) erstrecken und diese miteinander verbinden.

36. Baugruppe (1) nach Anspruch 34 oder 35, **dadurch gekennzeichnet, dass** der oder die Stützstege (49) in senkrechter Richtung zu den Stegen (41, 42) ausgerichtet sind.

37. Baugruppe (1) nach einem der Ansprüche 34 bis 36, **dadurch gekennzeichnet, dass** der oder die Stützstege (49) in senkrechter Richtung zur Längserstreckung des Kanals (35) ausgerichtet sind.

38. Baugruppe (1) nach einem der Ansprüche 34 bis 37, **dadurch gekennzeichnet, dass** der oder die Stützstege (49) ausgehend von der konkaven Vertiefung (48) im Bereich eines Kanalgrundes (44) des Kanals (35) enden.

39. Baugruppe (1) nach einem der Ansprüche 19 bis 38, **dadurch gekennzeichnet, dass** in der abdeckenden Stellung (Schutzstellung) des Schutzelementes (23) der abgedeckte Teil des Einstichelementes (3) im Bereich des Kanalgrundes (44) des Kanals (35) angeordnet ist.

40. Baugruppe (1) nach einem der Ansprüche 20 bis 39, **dadurch gekennzeichnet, dass** eine Kanaltiefe (50) des Kanals (35) in senkrechter Richtung zur Längserstreckung desselben ein Vielfaches, wie ein 5- bis 20-faches, einer äußeren Abmessung (51) des abzudeckenden Teils des Einstichelementes (3) beträgt.

41. Baugruppe (1) nach einem der Ansprüche 20 bis 40, **dadurch gekennzeichnet, dass** der Kanalgrund (44) im Bereich des Rückhalteelementes (36) eine Öffnung (52) aufweist.

42. Baugruppe (1) nach einem der Ansprüche 22 bis 41, **dadurch gekennzeichnet, dass** das Rückhalteelement (36) auf einem der Stege (41,42) innerhalb des Kanals (35) angeordnet ist und ausgehend von diesem in Richtung auf den Kanalgrund (44) in den Kanal (35) vorragt.

## Claims

1. Assembly (1), in particular for medical technology, comprising a manipulating device (2) with a first retaining element (11) and a first coupling element (12) disposed thereon, a puncturing element (3) with a proximal end and a distal end (13, 14) spaced apart from one another in the direction of a longitudinal axis (7) and a passage (15) connecting the two ends (13, 14) to one another, another coupling element (16) being disposed between the ends (13, 14), and the two coupling elements (12, 16) form a coupling mechanism (18), and the puncturing element (3) is mounted on the manipulating device (2), if necessary detachably, and the proximal end (13) of the puncturing element (3) extends out from the manipulating device (2), and an aperture (19) with a longer and a shorter opening axis (20, 21) is disposed at least on the proximal end (13), and with a pivotable guard device (4) for at least a part-region of the puncturing element (3), said guard device (4) being fixedly retained, if necessary detachably, on the first retaining element (11) of the manipulating device (2) by another retaining element (24), and the two retaining elements (11, 24) form a retaining mechanism (25), and a guard element (23) of the guard device (4) is designed to be pivotable in a pivot plane (22) extending in alignment with the longitudinal axis (7) from a first position (release position) releasing the part-region of the puncturing element (3) into a second position (guard position) covering said part-region, and a positioning device (26) is disposed between the two retaining elements (11, 24) of the retaining mechanism (25), which, when the retaining elements (11, 24) are engaged, fixes the position ofthe pivot plane (22) ofthe guard element (23) ofthe guard device (4) with respect to the manipulating device (2), and the two coupling elements (12, 16) of the coupling mechanism (18) are designed relative to one another in such a way that when the two coupling elements (12, 16) are in the fully engaged position, the shorter opening axis (21) of the aperture (19) is oriented approximately parallel with the pivot plane (22) or in the pivot plane (22), **characterised in that** the positioning device (26) comprises at least a first positioning element (33) oriented in the direction ofthe longitudinal axis (7) in the region of the manipulating device (2) and perpendicular to the latter, and at least one other positioning element (34) of an approximately complementary design in the region of the other retaining element (24).

2. Assembly (1) as claimed in claim 1, **characterised in that** the manipulating device (2) is formed by at least one receiving container (6) forming a receiving chamber (5) with an end face (8, 9) closed in at least certain regions by an end wall (10).

3. Assembly (1) as claimed in claim 1 or 2, **characterised in that** the first coupling element (12) and the first retaining element (11) are disposed concentrically with one another on the at least partially closed end face (9).

4. Assembly (1) as claimed in one of the preceding claims, **characterised in that** the first retaining element (11) is provided in the form of an approximately tubular first component (27).

5. Assembly (1) as claimed in claim 4, **characterised in that** the tubular component (27) extends out from the end wall (10) on the side facing away from the receiving chamber (5).

6. Assembly (1) as claimed in one of the preceding claims, **characterised in that** the other retaining element (24) is formed by another approximately tubular component (28) and it locates round the first tubular component (27) in at least certain regions around its external surface.

7. Assembly (1) as claimed in one of the preceding claims, **characterised in that** the retaining mechanism (25) further comprises catch elements (29, 30) which can be moved into mutual engagement and which are disposed on at least certain regions of the two retaining elements (11, 24).

8. Assembly (1) as claimed in claim 7, **characterised in that** the first catch element (29) on the first retaining element (11) is formed by a bead (31) extending out from the tubular component (27) on the side remote from the longitudinal axis (7) and extending around at least certain regions of the circumference.

9. Assembly (1) as claimed in claim 7 or 8, **characterised in that** the other catch element (30) on the other retaining element (24) is formed by another bead (32) extending out from the tubular component (28) on the side facing the longitudinal axis (7) and extending around at least certain regions of the circumference, and the other catch element (30) locates round the first catch element (29) on the side facing the end wall (10) in the latched position.

10. Assembly (1) as claimed in one of the preceding claims, **characterised in that** the guard element (23) is able to pivot about a pivot axis (37) oriented perpendicular to the pivot plane (22).

11. Assembly (1) as claimed in claim 10, **characterised in that** the pivot axis (37) is formed by a film hinge.

12. Assembly (1) as claimed in claim 11, **characterised in that** the film hinge forms a surmountable means of fixing the position of the guard element (23) before it is moved into the covering position (guard position) for the first time.

13. Assembly (1) as claimed in one of the preceding claims, **characterised in that** the coupling mechanism (18) is formed by an arrangement of threads engaging with one another.

14. Assembly (1) as claimed in claim 13, **characterised in that** the arrangement of threads is formed by a double-pitch thread.

15. Assembly (1) as claimed in one of the preceding claims, **characterised in that**, in a plane oriented perpendicular to the longitudinal axis (7), a stop component (38) of the other coupling element (16) facing the end wall (10) has a bigger dimension (39) than the internal dimension (40) of the other retaining element (24).

16. Assembly (1) as claimed in claim 1, **characterised in that** the first positioning element (33) is formed by a rib or a web.

17. Assembly (1) as claimed in claim 1 or 16, **characterised in that** the first positioning element (33) is disposed on the tubular component (27) of the first retaining element (11) and extends radially out from it towards the side remote from the longitudinal axis (7).

18. Assembly (1) as claimed in claim 1, **characterised in that** the other positioning element (34) is formed by a recess or a slot.

19. Assembly (1) as claimed in one of the preceding claims, **characterised in that** the guard element (23) of the guard device (4), is retained in a latched arrangement on the puncturing element (3) in the position in which it covers at least certain regions of the puncturing element (3).

20. Assembly (1) as claimed in one of the preceding claims, **characterised in that** the guard element (23) has a passage (35) for receiving at least a part-region of the puncturing element (3).

21. Assembly (1) as claimed in claim 20, **characterised in that** the passage (35) covers at least four sides of the puncturing element (3) to be covered in the covering position (guard position).

22. Assembly (1) as claimed in one of claims 19 to 21, **characterised in that** at least one retaining element (36) is disposed in the passage (35) for retaining the part-region of the puncturing element (3) latched in the guard position.

23. Assembly (1) as claimed in one of claims 19 to 22, **characterised in that**, when the guard element (23) is in the covering position (guard position), the retaining element (36) is disposed in the section between the other retaining element (24) of the guard device (4) and a half distance between the proximal end (13) and the other retaining element (24).

24. Assembly (1) as claimed in claim 23, **characterised in that** the retaining element (36) is disposed in about a third part of the total longitudinal extension of the passage (35), starting from the other retaining element (24).

25. Assembly (1) as claimed in one claims 20 to 24, **characterised in that** the passage (35) is bounded by an end wall (43) on the side remote from the other retaining element (24).

26. Assembly (1) as claimed in one of claims 20 to 25, **characterised in that** the passage (35) is bounded by webs (41, 42) extending parallel with one another across its longitudinal extension, spaced apart from one another perpendicular to the longitudinal extension.

27. Assembly (1) as claimed in claim 26, **characterised in that** the webs (41, 42) are spaced farther part from one another in the region close to the other retaining element (24) than in the rest of their longitudinal extension.

28. Assembly (1) as claimed in claim 26 or 27, **characterised in that** the two webs (41, 42) are connected to one another via the end wall (43) on the side remote from the other retaining element (24).

29. Assembly (1) as claimed in one of claims 20 to 28, **characterised in that** the passage (35) has an additional concave recess (48) between the retaining element (36) and the end wall (43).

30. Assembly (1) as claimed in one of claims 26 to 29, **characterised in that** a gripping bar (46, 47) extends out from at least one of the webs (41, 42) in the direction perpendicular to it on the side remote from the passage (35).

31. Assembly (1) as claimed in claim 30, **characterised in that** the gripping bars (46, 47) extend out from the two webs (41, 42) towards the side facing away from the passage (35).

32. Assembly (1) as claimed in claim 30 or 31, **characterised in that that** the gripping bar (46, 47) has a curved longitudinal contour across its longitudinal extension.

33. Assembly (1) as claimed in one of claims 30 to 32, **characterised in that** the gripping bar (46, 47) bounds the passage (35) in the region of the concave recess (48).

34. Assembly (1) as claimed in claim 29 or 33, **characterised in that** at least one supporting web (49) is disposed in the passage (35) in the region of the concave recess (48) for supporting and guiding the part of the puncturing element (3) to be received in the passage (35).

35. Assembly (1) as claimed in claim 34, **characterised in that** the one or more supporting g webs (49) extend between the two webs (41, 42) and connect the latter to one another.

36. Assembly (1) as claimed in claim 34 or 35, **characterised in that** the one or more supporting webs (49) are oriented in the direction perpendicular to the webs (41, 42).

37. Assembly (1) as claimed in one of claims 34 to 36, **characterised in that** the one or more supporting webs (49) are oriented in the direction perpendicular to the longitudinal extension of the passage (35).

38. Assembly (1) as claimed in one of claims 34 to 37, **characterised in that** the one or more supporting webs (49) terminate in the region of a passage base (44) of the passage (35), starting from the concave recess (48).

39. Assembly (1) as claimed in one of claims 19 to 38, **characterised in that** when the guard element (23) is in the covering position (guard position), the covered part of the puncturing element (3) is disposed in the region of the passage base (44) of the passage (35).

40. Assembly (1) as claimed in one of claims 20 to 39, **characterised in that** the depth (50) of the passage (35) in the direction perpendicular to the longitudinal extension thereof is a multiple, such as to 5 times to 20 times, an external dimension (51) of the part of the puncturing element (3) to be covered.

41. Assembly (1) as claimed in one of claims 20 to 40, **characterised in that** the passage base (44) of the passage (35) has an opening (52) in the region of the retaining element (36).

42. Assembly (1) as claimed in one of claims 22 to 41, **characterised in that** the retaining element (36) is disposed on one of the webs (41, 42) inside the passage (35) and extends out from it into the passage (35) in the direction towards the passage base (44).

## Revendications

1. Ensemble (1), en particulier pour la technique médicale, comprenant un dispositif de manipulation (2) avec un premier élément de retenue (11) disposé à celui-ci et avec un premier élément de couplage (12), un élément de piqûre (3) avec des extrémités proximale et distale (13, 14) qui sont espacées l'une de l'autre dans la direction d'un axe longitudinal (7), où un passage (15) relie les deux extrémités (13, 14) entre elles, et un autre élément de couplage (16) disposé entre les extrémités (13, 14), où les deux éléments de couplage (12, 16) forment un dispositif de couplage (18), et l'élément de piqûre (3) est retenu au dispositif de manipulation (2) selon le besoin amoviblement, où l'extrémité proximale (13) de l'élément de piqûre (3) fait saillie sur le dispositif de manipulation (2), et au moins à l'extrémité proximale (13) est ménagée une ouverture (19) avec des axes d'ouverture plus long et plus court (20, 21), un dispositif de protection pivotant (4) pour au moins une zone partielle de l'élément de piqûre (3), et le dispositif de protection (4) est retenu selon le besoin amoviblement avec un autre élément de retenue (24) d'une manière fixe au premier élément de retenue (11) du dispositif de manipulation (2), où les deux éléments de retenue (11, 24) forment un dispositif de retenue (25), et un élément de protection (23) du dispositif de protection (4) peut être pivoté dans un plan de pivotement (22) s'étendant dans l'axe longitudinal (7) d'une première position (position de libération) libérant la zone partielle de l'élément de piqûre (3) dans une deuxième position (position de protection) recouvrant la zone partielle, où est disposé entre les deux éléments de retenue (11, 24) du dispositif de retenue (25) un dispositif de positionnement (26) et celui-ci, lorsque les éléments de retenue (11, 24) sont en prise, déterminent le plan de pivotement (22) de l'élément de protection (23) du dispositif de protection (4) relativement au dispositif de manipulation (2) dans sa position, et en ce que les deux éléments de couplage (12, 16) du dispositif de couplage (18) sont réalisés l'un relativement à l'autre de façon que lors d'une position de couplage complète des deux éléments de couplage (12, 16), l'axe d'ouverture plus court (21) de l'ouverture (19) s'étend à peu près parallèlement au plan de pivotement (22) ou dans le plan de pivotement (22), **caractérisé en ce que** le dispositif de positionnement (26) est formé dans la zone du dispositif de manipulation (2) par au moins un premier élément de positionnement (33) orienté dans la direction de l'axe longitudinal (7) ainsi que perpendiculairement à celui-ci et, dans la zone de l'autre élément de retenue (24), par au moins un autre élément de positionnement (34) réalisé sensiblement d'une manière diamétralement opposée à celui-ci.

2. Ensemble (1) selon la revendication 1, **caractérisé en ce que** le dispositif de manipulation (2) est formé par au moins un contenant de réception (6) formant une chambre de réception (5) avec un côté frontal (8, 9) fermé au moins par zones par une paroi frontale (10).

3. Ensemble (1) selon la revendication 1 ou 2, **caractérisé en ce que** le premier élément de couplage (12) et le premier élément de retenue (11) sont disposés concentriquement l'un relativement à l'autre au côté frontal (9) fermé au moins par zones.

4. Ensemble (1) selon l'une des revendications précédentes, **caractérisé en ce que** le premier élément de retenue (11) est formé par un premier composant (27) sensiblement tubulaire.

5. Ensemble (1) selon la revendication 4, **caractérisé en ce que** le composant tubulaire (27) fait saillie sur la paroi frontale (10) au côté éloigné de la chambre de réception (5).

6. Ensemble (1) selon l'une des revendications précédentes, **caractérisé en ce que** l'autre élément de retenue (24) est formé par un autre composant à peu près tubulaire (28), et celui-ci passe sur le premier composant tubulaire (27) à sa surface externe au moins par zones.

7. Ensemble (1) selon l'une des revendications précédentes, **caractérisé en ce que** le dispositif de retenue (25) comprend en outre des éléments d'encliquetage (29, 30) pouvant être mis en prise qui sont disposés au moins par zones aux deux éléments de retenue (11, 24).

8. Ensemble (1) selon la revendication 7, **caractérisé en ce que** le premier élément d'encliquetage (29) est formé au premier élément de retenue (11) par un bourrelet (31) faisant saillie sur le composant tubulaire (27) sur le côté éloigné d'un axe longitudinal (7) et est disposé pour s'étendre au moins par zones sur le pourtour.

9. Ensemble (1) selon la revendication 7 ou 8, **caractérisé en ce que** l'autre élément d'encliquetage (30) est formé à l'autre élément de retenue (24) par un autre bourrelet (32) faisant saillie sur le composant tubulaire (28) au côté orienté vers l'axe longitudinal (7) et est disposé pour s'étendre au moins par zones sur le pourtour, et dans la position encliquetée, l'autre élément d'encliquetage (30) passe sur le premier élément d'encliquetage (29) au côté orienté vers la paroi frontale (10).

10. Ensemble (1) selon l'une des revendications précédentes, **caractérisé en ce que** l'élément de protection (23) peut être pivoté autour d'un axe de pivotement (37) orienté perpendiculairement au plan de pivotement (22).

11. Ensemble (1) selon la revendication 10, **caractérisé en ce que** l'axe de pivotement (37) est formé par une charnière de film.

12. Ensemble (1) selon la revendication 1 **caractérisé en ce que** la charnière de film, avant le premier déplacement dans la position de recouvrement (position de protection) forme une fixation de position surmontable pour l'élément de protection (23).

13. Ensemble (1) selon l'une des revendications précédentes, **caractérisé en ce que** le dispositif de couplage (18) est formé par un agencement de filetage en prise.

14. Ensemble (1) selon la revendication 13, **caractérisé en ce que** l'agencement de filetage est formé par un filetage à pas double.

15. Ensemble (1) selon l'une des revendications précédentes, **caractérisé en ce qu'**une partie de butée (38) orientée vers la paroi frontale (10) de l'autre élément de couplage (16) présente dans un plan orienté perpendiculairement à l'axe longitudinal (7) une plus grande dimension (39) par rapport à une dimension interne (40) de l'autre élément de retenue (24).

16. Ensemble (1) selon la revendication 1, **caractérisé en ce que** le premier élément de positionnement (33) est formé par une nervure ou arête.

17. Ensemble (1) selon la revendication 1 ou 16, **caractérisé en ce que** le premier élément de positionnement (33) est disposé au composant tubulaire (27) du premier élément de retenue (1 I) et fait saillie radialement sur celui-ci au côté éloigné de l'axe longitudinal (7).

18. Ensemble (1) selon la revendication 1, **caractérisé en ce que** l'autre élément de positionnement (34) est formé par un évidement ou une fente.

19. Ensemble (1) selon l'une des revendications précédentes, **caractérisé en ce que** l'élément de protection (23) du dispositif de protection (4), dans la position recouvrant l'élément de piqûre (3) au moins par zones, est retenu dans l'état encliqueté à l'élément de piqûre (3).

20. Ensemble (1) selon l'une des revendications précédentes, **caractérisé en ce que** l'élément de protection (23) présente un canal (35) pour la réception d'au moins une zone partielle de l'élément de piqûre (3).

21. Ensemble (1) selon la revendication 20, **caractérisé en ce que** le canal (35) recouvre sur au moins quatre côtés l'élément de piqûre à recouvrir (3) dans la position recouverte (position de protection).

22. Ensemble (1) selon l'une des revendications 19 à 21, **caractérisé en ce qu'**il est disposé dans le canal (35) au moins un élément de retenue (36) pour la retenue par encliquetage de la zone partielle de l'élément de piqûre (3) dans la position de protection.

23. Ensemble (1) selon l'une des revendications 19 à 22, **caractérisé en ce que** l'élément de retenue (36), dans la position de recouvrement (position de protection) de l'élément de protection (23), est disposé dans la section entre l'autre élément de retenue (24) du dispositif de protection (4) et à mi-distance entre l'extrémité proximale (13) et l'autre élément de retenue (24).

24. Ensemble (1) selon la revendication 23, **caractérisé en ce que** l'élément de retenue (36) est disposé à peu près dans le tiers de l'extension longitudinale totale du canal (35) en partant de l'autre élément de retenue (24).

25. Ensemble (1) selon l'une des revendications 20 à 24, **caractérisé en ce que** le canal (35) est délimité au côté éloigné de l'autre élément de retenue (24) par une paroi frontale (43).

26. Ensemble (1) selon l'une des revendications 20 à 25, **caractérisé en ce que** le canal (35) est délimité sur son extension longitudinale par des arêtes (41, 42) s'étendant parallèlement les unes aux autres et espacées perpendiculairement à l'extension longitudinale les unes des autres.

27. Ensemble (1) selon la revendication 26, **caractérisé en ce que** les arêtes (41, 42), dans la zone proche de l'autre élément de retenue (24), sont espacées davantage les unes des autres que dans leur extension longitudinale restante.

28. Ensemble (1) selon la revendication 26 ou 27, **caractérisé en ce que** les deux arêtes (41, 42), au côté éloigné de l'autre élément de retenue (24), sont reliées entre elles par la paroi frontale (43).

29. Ensemble (1) selon l'une des revendications 20 à 28, **caractérisé en ce que** le canal (35) présente entre l'élément de retenue (36) et la paroi frontale (43) un creux concave additionnel (48).

30. Ensemble (1) selon l'une des revendications 26 à 29, **caractérisé en ce qu'**au moins une des arêtes (41, 42), dans la direction perpendiculaire à celle-ci, est recouverte au côté éloigné du canal (35) par un rebord de prise (46, 47).

31. Ensemble (1) selon la revendication 30, **caractérisé en ce que** les rebords de prise (46, 47) font saillie sur les deux arêtes (41, 42) au côté éloigné du canal (35).

32. Ensemble (1) selon la revendication 30 ou 31, **caractérisé en ce que** le rebord de prise (46, 47) présente sur son extension longitudinale une courbe longitudinale.

33. Ensemble (1) selon l'une des revendications 30 à 32, **caractérisé en ce que** le rebord de prise (45, 46) délimite le canal (35) dans la zone du creux concave (48).

34. Ensemble (1) selon la revendication 29 ou 33, **caractérisé en ce qu'**il est disposé dans le canal (35) dans la zone du creux concave (48) au moins une arête d'appui (49) pour l'appui et le guidage de la partie de l'élément de piqûre (3) à recevoir dans le canal (35).

35. Ensemble (1) selon la revendication 34, **caractérisé en ce que** la ou les arêtes d'appui (49) s'étendent entre les deux arêtes (41, 42) et relient celles-ci entre elles.

36. Ensemble (1) selon la revendication 34 ou 35, **caractérisé en ce que** la ou les arêtes d'appui (49) sont orientées dans la direction perpendiculaire aux arêtes (41, 42).

37. Ensemble (1) selon l'une des revendications 34 à 36, **caractérisé en ce que** la ou les arêtes d'appui (49) sont orientées dans la direction perpendiculaire à l'extension longitudinale du canal (35).

38. Ensemble (1) selon l'une des revendications 34 à 37, **caractérisé en ce que** la ou les arêtes d'appui (49), en partant du creux concave (48), se terminent dans la zone d'un fond (44) du canal (35).

39. Ensemble (1) selon l'une des revendications 19 à 38, **caractérisé en ce que** dans la position de recouvrement (position de protection) de l'élément de protection (23), la partie recouverte de l'élément de piqûre (3) est disposée dans la zone du fond (44) du canal (35).

40. Ensemble (1) selon l'une des revendications 20 à 39, **caractérisé en ce qu'**une profondeur (50) du canal (35) dans la direction perpendiculaire à l'extension longitudinale de celui-ci représente un multiple, par exemple 5 à 20 fois, d'une dimension extérieure (51) de la partie à recouvrir de l'élément de piqûre (3).

41. Ensemble (1) selon l'une des revendications 20 à 40, **caractérisé en ce que** le fond de canal (44) présente dans la zone de l'élément de retenue (36) une ouverture (52).

42. Ensemble (1) selon l'une des revendications 22 à 41, **caractérisé en ce que** l'élément de retenue (36) est disposé sur l'une des arêtes (41, 42) à l'intérieur du canal (35) et, en partant de celui-ci, fait saillie en direction du fond (44) dans le canal (35).
